# EUROPEAN PATENT APPLICATION

(11) **EP 2 843 025 A1**
(43) Date of publication of application: **04.03.2015**
(21) Application number: 13782338.1
(22) Date of filing: 17.04.2013
(51) Int. Cl.: C09K 9/02, C07C 231/02, C07C 233/36, C07C 233/42, C07C 233/69, C08F 2/44, C09D 4/06, C09D 5/29

(54) **PHOTOCHROMIC CURABLE COMPOSITION**

(30) Priority: 27.04.2012 JP 2012102850
(71) Applicant: Tokuyama Corporation, Shunan-shi, Yamaguchi-ken 745-8648 (JP)
(72) Inventor: IZUMI, Shinobu, Shunan-shi Yamaguchi 745-8648 (JP); TAKENAKA, Junji, Shunan-shi Yamaguchi 745-8648 (JP); MOMODA, Junji, Shunan-shi Yamaguchi 745-8648 (JP)
(74) Representative: Duckworth, Timothy John
(86) International application number: PCT/JP2013/061402
(87) International publication number: WO 2013/161642

(57) **Abstract**

A photochromic curable composition containing, as a radically polymerizable monomer component (A), a (meth)acrylic - amide polymerizable monomer (A1) having 2, to 6 (meth)acryloyloxy groups and 2 to 6 divalent amide groups (-NHCO-), and a non-amide polymerizable monomer (A2) having at least 3 radically polymerizable groups but having no amide bond. From the photochromic composition, there can be obtained a photochromic cured body having excellent photochromic properties such as a high color density, a high fading rate, and excellent mechanical strength.

## Description

### Technical Field:

This invention relates to a novel photochromic curable composition that can be preferably used for the production of a photochromic cured body having excellent photochromic action.

### Background Art:

Photochromism is a reversible action of a compound which, when irradiated with light containing ultraviolet rays such as sunlight or light of a mercury lamp, quickly changes its color and, when no longer irradiated with light and placed in a dark place, resumes its original color, and has now been applied to the use in a variety of fields. As photochromic compounds having such properties, there have been known fulgimide compounds, spirooxazine compounds and chromene compounds. Upon being compounded with a plastic material, these compounds can be formed into optical articles having photochromic properties, and study has been forwarded extensively for compounding them together.

For instance, photochromism has been applied in the field of spectacle lenses, too. When irradiated with light containing ultraviolet rays such as of sunlight outdoors, the photochromic spectacle lenses using a photochromic compound quickly develop color and work as sunglasses. In the indoors where there is no irradiation of such light, the photochromic spectacle lenses have faded color and work as ordinary transparent spectacles. In recent years, demands are increasing for the photochromic spectacle lenses.

As the photochromic spectacle lenses, in particular, plastic lenses have been desired from the standpoint of small weight and safety. To impart the plastic lenses with photochromic properties, in general, the plastic lenses are compounded with the photochromic compound. As the compounding methods, there have been known an imbibition method, an in-mass method and a coating method.

The imbibition method is a method of imbibing the surfaces of the lenses with the photochromic compound (see, for example, patent documents 1 to 3).

The in-mass method is a method of dissolving a photochromic compound in a monomer so as to be polymerized to directly obtain photochromic lenses (see, for example, patent documents 4 to 6). This method has an advantage of inexpensively producing in large quantity the photochromic lenses by utilizing the glass molding. Therefore, most of the photochromic plastic lenses have now been produced by this method.

The coating method is a method of forming a coating (photochromic coating) having photochromic properties on the surfaces of the plastic lenses (see, for example, patent documents 7 and 8). The coating method maintains the mechanical strength of the plastic lenses themselves and, therefore, enables the photochromic coating to be designed relatively freely making it easy to obtain photochromic lenses (optical materials) having excellent photochromic properties and, specifically, quick color fading rate.

Here, the following properties are required for the photochromic compounds and for the photochromic plastic optical articles containing photochromic compounds:
(a) The colored degree (initial color) is low in the visible ray region of before being irradiated with ultraviolet rays;
(b) The colored degree (color density) is high when irradiated with ultraviolet rays;
(c) The rate of return (fading rate) is high from when the irradiation with ultraviolet rays is discontinued until when the initial state is resumed;
(d) The reversible action of developing color - fading color has good recurring durability;
(e) Storage stability is high;
(f) Can be easily formed into optical articles; and
(g) The optical articles have a large mechanical strength.

With the above art as a background, there has been proposed photochromic plastic lenses (optical materials) using a chromene compound that is little subject to be decomposed by light and that permits a little decrease in its color-developing capability even after it is continuously irradiated with sunlight or light resembling sunlight. Among them, photochromic curable compositions have been developed combining various polymerizable monomers with photochromic compounds (specifically, chromene compounds) relying upon the in-mass method and the coating method.

For instance, patent documents 4 to 6 are disclosing photochromic curable compositions using specific (meth)acrylic polymerizable monomers that have a (meth)acrylic group in combination with chromene compounds.

However, the photochromic curable compositions described in the patent documents 4 to 6 have a problem in that the mechanical strength of the obtained cured bodies can be maintained but at least sacrificing photochromic properties.

On the other hand, patent documents 7 and 8 are disclosing photochromic curable compositions obtained by combining a closely adhering component such as a compound having a silanol group, a compound having an isocyanate group or an amine compound with a radically polymerizable monomer and a chromene compound. These curable compositions are adapted to be used in the coating method and provide an advantage of forming a photochromic coating having excellent adhesiveness to the plastic lenses.

With the photochromic curable compositions disclosed in the patent documents 7 and 8, however, there still remains room for improvement in regard to that the obtained cured bodies (i.e., photochromic coatings) have a low hardness. Namely, the photochromic coating having a low hardness is often scratched in the step of polishing the back surfaces of the lenses for gaining a desired degree or in the step of working the lenses such as shaping the circumferential edges of the lenses to meet the shape of the frame, causing a decrease in the productivity.

### Prior Art Documents:

### Patent Documents:

Patent document 1: USP 5739243
Patent document 2: USP 5973093
Patent document 3: WO95/10790
Patent document 4: WO01/005854
Patent document 5: WO04/083268
Patent document 6: WO09/075388
Patent document 7: WO03/011967
Patent document 8: WO05/014717

### Outline of the Invention:

### Problems that the Invention is to Solve:

It is, therefore, an object of the present invention to provide a photochromic curable composition capable of forming a cured body which excels in both photochromic properties and mechanical strength.

### Means for Solving the Problems:

In order to solve the above problems, the present inventors have conducted keen study. As a result, the inventors have discovered that a curable composition capable of forming a cured body having photochromic properties and mechanical strength can be obtained upon combining, as a radically polymerizable monomer component, a (meth)acrylic polymerizable monomer having 2 to 6 (meth)acryloyloxy groups and 2 to 6 divalent amide groups with a non-amide polymerizable monomer having at least 3 radically polymerizable groups, and have completed the invention.

That is, according to the present invention, there is provided a photochromic curable composition containing a radically polymerizable monomer component (A) and a photochromic compound (B), wherein the radically polymerizable monomer component (A) contains:
(A1) a (meth)acrylic - amide polymerizable monomer having 2 to 6 (meth)acryloyloxy groups and 2 to 6 divalent amide groups (-NHCO-); and
(A2) a non-amide polymerizable monomer having at least 3 radically polymerizable groups but having no amide bond.

In the photochromic composition of the present invention, the following embodiments (1) to (6) are desired:
(1) The (meth)acrylic - amide polymerizable monomer (A1) is represented by the following general formula (1): 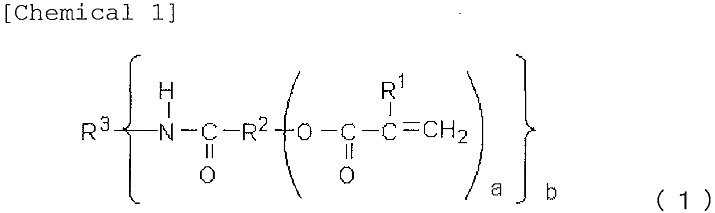 wherein,
   a is an integer of 1 to 3 and b is an integer of 2 to 6 provided the product of a and b is an integer of 2 to 6,
   R¹ is a hydrogen atom or a methyl group,
   R² is an organic group having a valency of (a+1),
      and
   R³ is an organic group having a valency of (b), or by the following general formula (2): 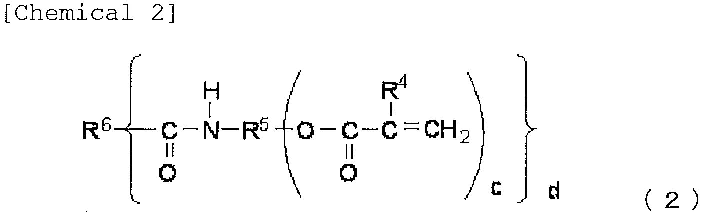 wherein,
   c is an integer of 1 to 3 and d is an integer of 2 to 6 provided the product of c and d is an integer of 2 to 6,
   R⁴ is a hydrogen atom or a methyl group,
   R⁵ is an organic group having a valency of (c+1),
      and
   R⁶ is an organic group having a valency of (d).
(2) The non-amide polymerizable monomer (A2) is a radically polymerizable monomer represented by the following general formula (3): 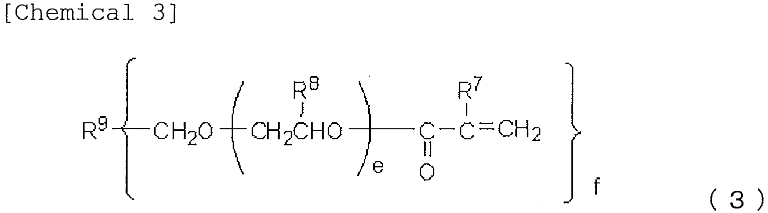 wherein,
   e is an integer of 0 to 3,
   f is an integer of 3 to 6,
   R⁷ and R⁸ are hydrogen atoms or methyl groups,
      and
   R⁹ is an organic group having a valency of (f).
(3) The non-amide polymerizable monomer (A2) has an average composition represented by the following unit formula (4):

   R¹⁰SiO_{3/2} (4)

   wherein,
   R¹⁰ is a group bonded to a silicon atom, and is an organic group containing hydrogen atom, alkyl group, cycloalkyl group, alkoxy group, halogenated alkyl group, phenyl group, halogen-substituted phenyl group, hydroxyl group or radically polymerizable group,
   and in a molecule thereof, at least 3 groups R¹⁰ are organic groups containing a radically polymerizable group, and have a polymerization degree of 6 to 100.
(4) The photochromic compound (B) contains a compound that has an indeno[2,1-f]naphtho[1,2-b]pyran skeleton.
(5) The polymerizable monomer component (A) contains the (meth)acrylic - amide polymerizable monomer (A1) in an amount of 10 to 80% by mass, the non-amide polymerizable monomer (A2) in an amount of 3 to 50% by weight and the non-amide polymerizable monomer (A3) other than the component (A2) in an amount of 0 to 87% by mass.
(6) The photochromic compound (B) is contained in an amount of 0.001 to 20 parts by mass per 100 parts by mass of the polymerizable monomer component (A).

According to the present invention, further, there is provided a photochromic coating agent comprising a photochromic cured body obtained by curing the photochromic curable composition or comprising the photochromic curable composition.

According to the present invention, further, there is provided a photochromic optical material by coating the surfaces of an optical material with a photochromic coating that is obtained by curing the photochromic coating agent.

The (meth)acrylic - amide polymerizable monomer represented by the above general formula (1) is a novel compound.

In the (meth)acrylic - amide polymerizable monomer, it is desired that the group R² in the general formula (1) is a group represented by the following formula (1a) or (1b), 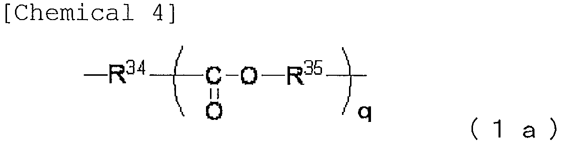 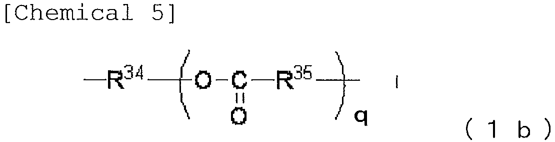 wherein in the formulas (1a) and (1b),
R³⁴ is an organic group having a valency of (a+1) that is bonded to a carboxyl oxygen atom in the (meth)acryloyloxy group, and is a group in which a saturated hydrocarbon group or a hydrogen atom in part of the saturated hydrocarbon group is substituted with an alkoxy group or a phenoxy group,
or is a group represented by the following formula (1ab-1) : 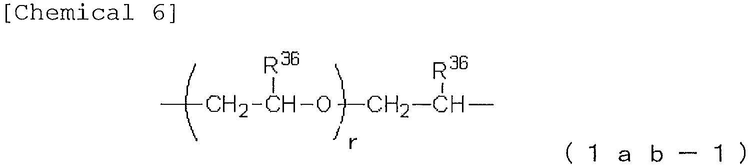
(wherein R³⁶ is a hydrogen atom or a methyl group, and r is an integer of 0 to 20),
R³⁵ is a divalent organic group, and is a saturated hydrocarbon group, an alicyclic group or an aromatic hydrocarbon group, and
q is an integer of 1 to 30.

In the above general formula (1), further, it is desired that the group R³ is a group having an aromatic ring, a group having an alicyclic ring, or a saturated hydrocarbon group provided the total number of carbon atoms is 1 to 30, or that these groups are oxygen-containing groups coupled to each other via -CO-, -COO- or -O-.

The (meth)acrylic - amide polymerizable monomer of the above general formula (1) is produced by reacting a carboxylic acid derivative represented by the following formula (1X): 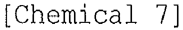 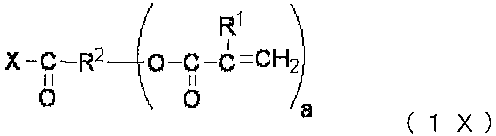 wherein,
R¹, R² and a are as defined in the above formula (1),
X is a hydroxyl group, a chlorine atom, a bromine atom, -N₃ or a group represented by the following formula:

   R³⁷-COO-
(wherein R³⁷ is an alkyl group or an aryl group), with an amine compound represented by the following formula (1Y) :

   R³-(NH₂)_{b} (1Y)
wherein R³ and b are as defined in the above formula (1),
in a mixed solvent of an organic solvent and water in the presence of a basic substance.

### Effects of the Invention:

The photochromic composition of the present invention has a distinguished feature in the use, as the radically polymerizable monomer (A), of a (meth)acrylic - amide polymerizable monomer (A1) that has 2 to 6 (meth)acryloyloxy groups and 2 to 6 divalent amide groups in combination with a non-amide polymerizable monomer (A2) that has at least 3 radically polymerizable groups.

Namely, the non-amide polymerizable monomer (A2) works as a crosslinking monomer, and a photochromic curable composition that uses the crosslinking monomer has heretofore been widely known. If it is attempted to obtain a highly strong cured body by using the crosslinking monomer only, however, the cured body becomes too dense impairing photochromic properties and exhibiting, for example, greatly decreased fading rate. This is caused by a greatly limited freedom of molecules of the photochromic compound.

If, however, the photochromic compound is combined with the above (meth)acrylic - amide polymerizable monomer (A1) according to the present invention, then it is allowed to obtain a cured body having a large mechanical strength without impairing photochromic properties as demonstrated in Examples appearing later.

In the present invention, it was discovered as a phenomenon through extensive experiments that by using the (meth)acrylic - amide polymerizable monomer (A1) in combination, the cured body exhibits both photochromic property and mechanical strength though the reason has not yet been clarified. The inventors are speculating that a hydrogen atom in the amide group possessed by the (meth)acrylic - amide polymerizable monomer (A1) is presumably forming a hydrogen bond with an oxygen atom in the polymer chain. Namely, it is considered that the hydrogen bond works to form a loosely and falsely crosslinked structure in addition to the crosslinked structure that is formed by the ordinary radical polymerization and, as a result, it is allowed to obtain a cured body having a high density and a large mechanical strength without impairing the freedom of molecules of the photochromic compound. According to the present invention as will be understood, for example, from Examples described later, a particularly high fading rate is obtained yet assuring a large mechanical strength proving the fact that the freedom of molecules of the photochromic compound has not been impaired and that a falsely crosslinked structure has been formed without impairing the freedom of molecules.

According to the present invention as described above, it is allowed to obtain a photochromic cured body exceling in photochromic properties and mechanical strength.

The photochromic curable composition of the invention can be used for obtaining photochromic formed articles by the in-mass method, and is very useful as a photochromic coating agent for forming a photochromic coating having excellent strength on the surfaces of the optical materials.

### Modes for Carrying Out the Invention:

The photochromic curable composition of the present invention contains the radically polymerizable monomer component (A) and the photochromic compound (B) and, further, contains, as required, a suitable blending agent (C).

The components will now be described in detail.

### <Radically polymerizable monomer component (A)>

The present invention uses, as the radically polymerizable monomer component(A), a (meth)acrylic - amide polymerizable monomer (A1) and a non-amide polymerizable monomer (A2) having at least 3 radically polymerizable groups, and, further, uses, as required, a non-amide polymerizable monomer (A3) other than (A2).

### 1. (Meth)acrylic - amide polymerizable monomer (A1):

The (meth)acrylic - amide polymerizable monomer (A1) is an essential component of the photochromic curable composition of the present invention, and has 2 to 6 (meth)acryloyloxy groups and 2 to 6 divalent amide groups (-NHCO-). As described above, upon using the polymerizable monomer (A1) in combination with a non-amide polymerizable monomer (A2) that will be described later, it is allowed to obtain a photochromic cured body exceling in both photochromic property and mechanical strength.

That is, the (meth)acyloyloxy group is represented by the formula:

CH₂ = C(R) - COO -

wherein R is a hydrogen atom or a methyl group.

Due to the polymerization based on the unsaturated bonding of the above group, molecules of the (meth)acrylic - amide polymerizable monomer (A1) are incorporated in the polymer chain formed by the non-amide polymerizable monomer (A2) that will be described later.

If the number of the (meth)acryloyloxy groups in the (meth)acrylic - amide polymerizable monomer (A1) is not less than 6, it is allowed to obtain a cured body having a large mechanical strength. In this case, however, the crosslinked structure becomes so dense that the cured body loses the photochromic property. If the number of the (meth)acryloyloxy group is one, on the other hand, the polymerizable monomer (A1) is not incorporated in a sufficiently large amount in the crosslinked structure. As a result, though the cured body may assure photochromic properties, its mechanical strength becomes unsatisfactory.

In the present invention, two is the optimum number of the (meth)acryloyloxy groups in the (meth)acrylic - amide polymerizable monomer (A1).

In the present invention, further, the divalent amide group (-NHCO-) is also called peptide bond. As described earlier, the amide group is present in the polymerizable monomer (A1) and, therefore, it is presumed that the hydrogen atom in the amide group forms a hydrogen bond with an oxygen atom in the polymer chain formed by the non-amide polymerizable monomer (A2) that will be described later or formed by the polymerizable monomer (A1) thereof. As a result, a loose and easily stretchable falsely crosslinked structure is formed in the cured body together with the ordinary rigid crosslinked structure making it, therefore, possible to obtain a photochromic cured body having a large mechanical strength without impairing photochromic properties.

For example, if the hydrogen atom of the amide group has been substituted, there is formed no falsely crosslinked structure by the hydrogen bond and the mechanical strength becomes unsatisfactory. Further, if the non-amide polymerizable monomer (A2) that will be described later is used in too large amounts so as to obtain large mechanical strength, freedom of the photochromic compound is lost and the photochromic properties (specifically, fading rate) are impaired.

Further, if the amide group present in the polymerizable monomer (A1) is monovalent, i.e., (-OCNH₂), the falsely crosslinked structure is densely formed. Therefore, though the mechanical strength of the cured body can be improved, freedom of the photochromic compound is lost whereby the photochromic properties decrease and, for instance, the fading rate decreases.

From the above point of view according to the present invention, the polymerizable monomer (A1) must possess divalent amide groups in a number of 2 to 6. That is, if the number of the amide groups is more than 6, then the falsely crosslinked structure is densely formed by the hydrogen bond and the photochromic properties become inferior. Further, if the number of the amide group is 1, the falsely crosslinked structure is not sufficiently formed by the hydrogen bond, and the mechanical strength of the cured body decreases.

The above-mentioned (meth)acrylic - amide polymerizable monomer should have (meth)acryloyloxy groups and amide groups (-HNCO-) in a number of 2 to 6, respectively. Preferably, there can be used the (meth)acrylic - amide polymerizable monomer (A1-1) represented by the general formula (1) or the (meth)acrylic - amide polymerizable monomer (A1-2) represented by the general formula (2) and, most desirably, the (meth)acrylic - amide polymerizable monomer (A1-1) represented by the general formula (1).

### (Meth)acrylic - amide polymerizable monomer (A1-1):

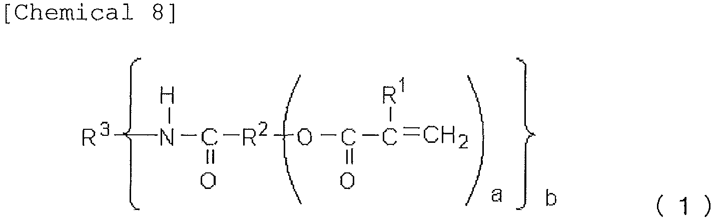

wherein,
a is an integer of 1 to 3 and b is an integer of 2 to 6 provided the product of a and b is an integer of 2 to 6,
R¹ is a hydrogen atom or a methyl group,
R² is an organic group having a valency of (a+1), and
R³ is an organic group having a valency of (b).

The (meth)acrylic - amide polymerizable monomer (A1-1) has a structure in which the (meth)acryloyloxy group is bonded to the amide group on the side of the carbonyl group (C=O), and is most desirably used in the present invention.

### (Meth)acrylic - amide polymerizable monomer (A1-2):

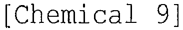

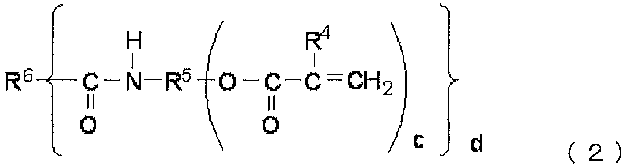

wherein,
c is an integer of 1 to 3 and d is an integer of 2 to 6 provided the product of c and d is an integer of 2 to 6,
R⁴ is a hydrogen atom or a methyl group,
R⁵ is an organic group having a valency of (c+1), and
R⁶ is a organic group having a valency of (d).

Contrary to the above monomer (A1-1), the (meth)acrylic - amide polymerizable monomer (A1-2) has a structure in which the (meth)acryloyloxy group is bonded to the amide group on the side of the nitrogen atom.

### (a, b, c and d).

In the above general formulas (1) and (2), a and C are both integers of 1 to 3, and (a+1) and (c+1) correspond to valencies of the group R² and the group R⁵.

Further, b and d correspond to valencies of the group R³ or the group R⁶, and represent the number of the divalent amide groups. Therefore, both b and d must be integers within a range of 2 to 6.

Further, both the product (a·b) of a and b and the product (c·d) of c and d represent the numbers of the (meth)acryloyloxy groups. Therefore, their products must be 2 to 6.

In the invention, it is desired that b and d are integers of 2 to 3 from such a standpoint that the cured body that is obtained has a large mechanical strength and, specifically, are 2 from the standpoint of productivity of the monomers. It is, further desired that a and c are 1. Namely, most desirably, both the (meth)acrylic - amide polymerizable monomer (A1-1) of the general formula (1) and the (meth)acrylic - amide polymerizable monomer (A1-2) of the general formula (2), have two amide groups and two (meth)acryloyloxy groups.

### (Group R¹ and group R⁴)

R¹ in the general formula (1) and R⁴ in the general formula (2) are both hydrogen atoms or methyl groups. Namely, the polymerizable monomers of the general formulas (1) and (2) both have the acryloyloxy group (R¹, R⁴ = hydrogen atoms) or the methacryloyloxy group (R¹, R⁴ = methyl groups).

### (Group R² and group R⁵)

R² in the general formula (1) and R⁵ in the general formula (2) are organic groups having valencies of 2 to 4 as will be learned from that a and c are integers of 1 to 3.

As the organic groups having valencies of 2 to 4, there can be exemplified hydrocarbon groups and groups (oxygen-containing hydrocarbon groups) in which hydrocarbon groups are bonded together via a divalent bridging group (e.g., -CO-, -COO-, -O-) having an oxygen atom.

The hydrocarbon group has carbon atoms in a total number in a range of, for example, 1 to 30, and its concrete examples include alkylene groups such as methylene group, ethylene group, n-propylene group, isopropylene group, n-butylene group, n-pentylene group, and n-hexylene group; cycloalkylene groups such as cyclohexylene group; and arylene groups such as phenylene group and naphthylene group.

In the present invention, it is desired that the group R² in the general formula (1) and the group R⁵ in the general formula (2) are the above-mentioned oxygen-containing groups. As the oxygen-containing groups, there can be exemplified those represented by the following formula (1a) or (1b): 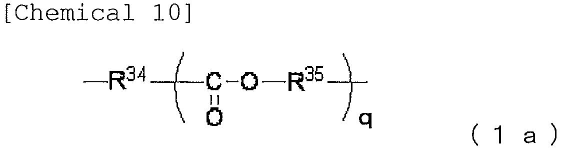 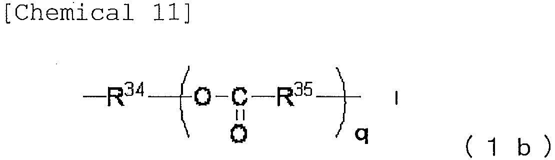 wherein in the formulas (1a) and (1b),
R³⁴ is an organic group having a valency of 2 to 4 that is bonded to the carboxyl oxygen atom in the (meth)acryloyloxy group, and is a saturated hydrocarbon group or a group (substituted and saturated hydrocarbon group) in which a hydrogen atom of a part of the saturated hydrocarbon group is substituted with an alkoxy group or a phenoxy group, or is a divalent group represented by the following formula (1ab-1): 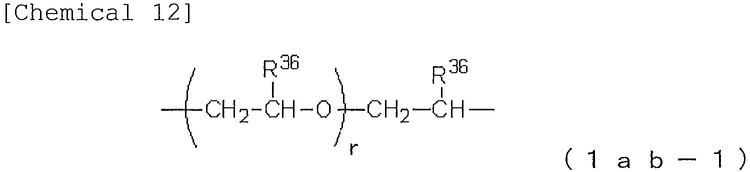 (wherein R³⁶ is a hydrogen atom or a methyl group, and r is an integer of 0 to 20),
R³⁵ is a divalent organic group, and is a saturated hydrocarbon group, an alicyclic group or an aromatic hydrocarbon group, and
q is an integer of 1 to 30.

The groups represented by the above formulas (1a) and (1b) both have an ester structure, but the two are different from each other only in regard to that the oxygen atoms are bonded to the C=O group forming the ester at positions opposite to each other.

Here, the ester structure in the formula (1b) can be derived from the hydroxyl group-containing monomer enabling various kinds of starting materials to be easily obtained as compared to those having the ester structure in the formula (1a). Therefore, it is allowed to easily produce the polymerizable monomers (A1-1) and (A1-2) having properties that meet the objects.

In the above formulas (1a) and (1b), q is an integer of 1 to 30. From the standpoint of obtaining a cured body having a high hardness by polymerizing the monomer, however, it is desired that q is an integer of 1 to 20 and, specifically, 1 to 10.

It is desired that the saturated hydrocarbon group (having a valency of 2 to 4) which is the group R³⁴ is a straight-chain or branched saturated hydrocarbon group having 1 to 10 carbon atoms.

As the saturated hydrocarbon group, there can be exemplified divalent groups such as methylene group, ethylene group, trimethylene group, propylene group, tetramethylene group, isobutylene group, pentamethylene group and hexamethylene group, as well as trivalent and tetravalent groups represented by the following formulas:

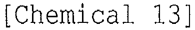

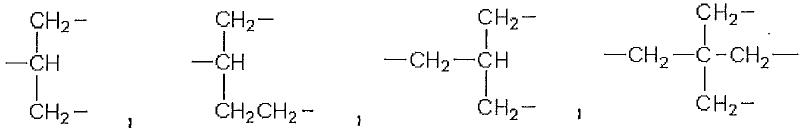

Further, the substituted and saturated hydrocarbon group which is the group R³⁴ is the one having an alkoxy group or a phenoxy group as a substituent and, specifically, is the above-mentioned saturated hydrocarbon group having 1 to 10 carbon atoms and to which an alkoxy group or a phenoxy group is bonded as a substituent.

It is desired that the substituted and saturated hydrocarbon group has the alkoxy groups or the phenoxy groups which are the substituents in a number of 1 to 5 and, specifically, 1 to 3.

The alkoxy group desirably has 1 to 5 carbon atoms, and preferred examples thereof are methoxy group and ethoxy group.

In the divalent group represented by the formula (1ab-1) which is the group R³⁴, r is an integer of 0 to 20. From the standpoint of obtaining a cured body having a high hardness by polymerizing the monomer, however, it is desired that r is an integer of 0 to 15 and, more desirably, 0 to 10.

Among the above groups R³⁴, specifically desired are alkylene groups such as methylene group, ethylene group, trimethylene group, tetramethylene group and propylene group, as well as the groups represented by the following formulas: 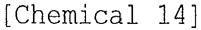 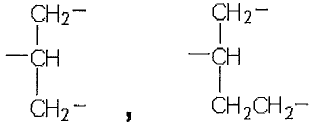 from the standpoint of productivity and yields of the (meth)acrylic - amide polymerizable monomers (A1-1) and (A1-2).

In the formulas (1a) and (1b) representing the group R² or the group R⁵, the group R³⁵ is a divalent saturated hydrocarbon group, a divalent alicyclic group or a divalent aromatic hydrocarbon group.

The divalent saturated hydrocarbon group is, preferably, a straight-chain or branched alkylene group having 1 to 20 carbon atoms and, more preferably, a straight-chain or branched alkylene group having 1 to 6 carbon atoms. From the standpoint of easy availability of the starting material, the straight-chain alkylene group having 1 to 6 carbon atoms is preferred. Concretely, most preferred are methylene group, ethylene group, trimethylene group, tetramethylene group, pentamethylene group and hexamethylene group.

As the divalent alicyclic group, a cycloalkylene group having 3 to 20 carbon atoms is preferred and a cycloalkylene group having 4 to 12 carbon atoms is more preferred. From the standpoint of easy availability of the starting material, the cycloalkylene group having 5 to 8 carbon atoms is most preferred, such as cyclobutylene group, cyclopentylene group, cyclohexylene group, cycloheptylene group and cyclooctylene group.

It is desired that the divalent aromatic hydrocarbon group has 6 to 20 carbon atoms and, further, has an aromatic hydrocarbon ring such as benzene ring, naphthalene ring or biphenyl ring. Among them, most preferred are 2-phenylene group, 1,3-phenylene group, 1,4-phenylene group, 1,2-naphthylene group, 1,4-naphthylene group, 1,8-naphthylene group, 2,3-naphthylene group, 2,6-naphthylene group, 2,2'-biphenylene group and 4,4'-biphenylene group from the standpoint of easy availability of the starting materials.

In the invention, the oxygen-containing groups most preferred as the group R² in the general formula (1) and as the group R⁵ in the general formula (2) are those exemplified below.

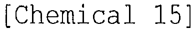

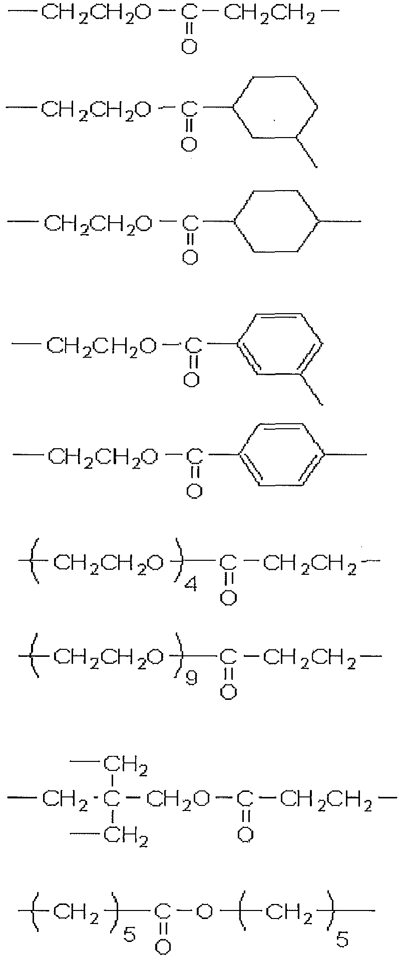

### (Group R³ and group R⁶)

In the general formula (1) or (2), the groups R³ and R⁶ are organic groups having a valency (concretely, a valency of 2 to 6) that corresponds to b or d in the formulas.

Though there is no specific limitations, the organic groups having valencies of 2 to 6 are, preferably, those having carbon atoms in a total number of 1 to 30 and are, specifically, hydrocarbon groups having an aromatic ring, hydrocarbon groups having an alicyclic ring, or acyclic saturated hydrocarbon groups. It is, further, desired that these groups are the groups (oxygen-containing hydrocarbon groups) that are bonded together via a divalent bridging group (e.g., -CO-, -COO-, -O-) having an oxygen atom provided the total number of the carbon atoms is 1 to 30.

### Hydrocarbon groups (R³, R⁶) having an aromatic ring:

As the aromatic ring possessed by the hydrocarbon group having a valency of 2 to 6, there can be exemplified a benzene ring and a naphthalene ring. The aromatic ring is not limited to a single ring but may be of such a form in which a plurality of rings are bonded together directly or via an alkyene group such as methylene group like the biphenyl ring. Further, the aromatic ring may have a substituent bonded thereto. The bonding hand may be present on the aromatic ring or on a coupling group such as methylene group or on a substituent of the aromatic ring.

Preferred examples of the hydrocarbon group having the aromatic ring are the divalent groups represented by the following formulas: 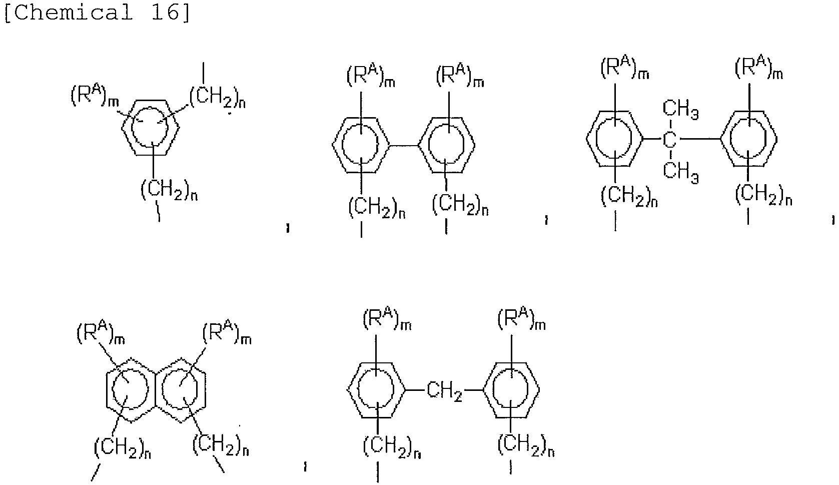 wherein R^{A} is an alkyl group having 1 to 4 carbon atoms or an alkoxy group having 1 to 4 carbon atoms, m represents the number of the substituents R^{A} and is an integer of 0 to 3, and n represents the number of chains of methylene and is an integer of 0 or 1.

In the above formula, n=0 means that the bonding hand is present on the aromatic ring.

Among the divalent groups having the aromatic ring, specifically preferred are those represented by the following formulas:

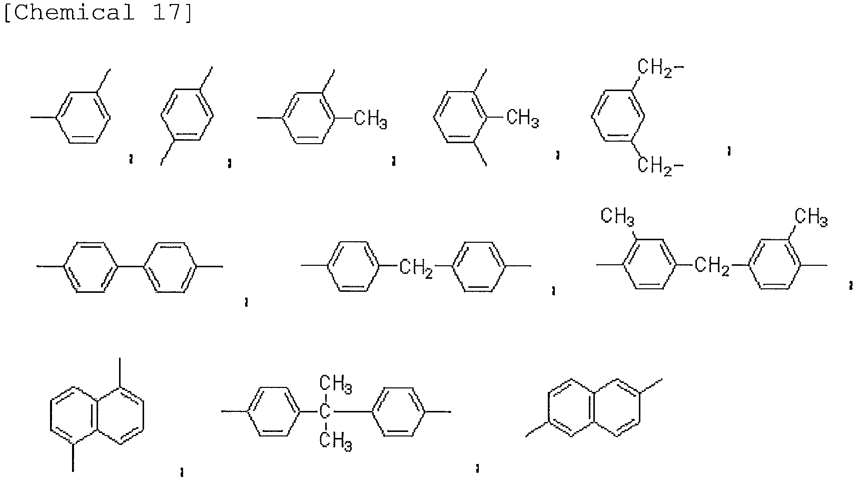

### Hydrocarbon groups (R³, R⁶) having an aliphatic ring:

As the aliphatic ring possessed by the hydrocarbon group having a valency of 2 to 6, there can be exemplified a cyclo ring and a bicyclo ring. The aliphatic ring is not limited to a single ring but may be of such a form in which a plurality of rings are bonded together directly or via an alkyene group such as methylene group like the hydrogenated biphenyl and the hydrogenated diphenylmethane. Further, the aliphatic ring may have a substituent bonded thereto. The bonding hand may be present on the aliphatic ring or on a coupling group such as methylene group or on a substituent of the aliphatic ring.

Among the hydrocarbon groups having an aliphatic group and a valency of 2 to 6, preferred are the divalent hydrocarbon groups as concretely represented by the following formulas: 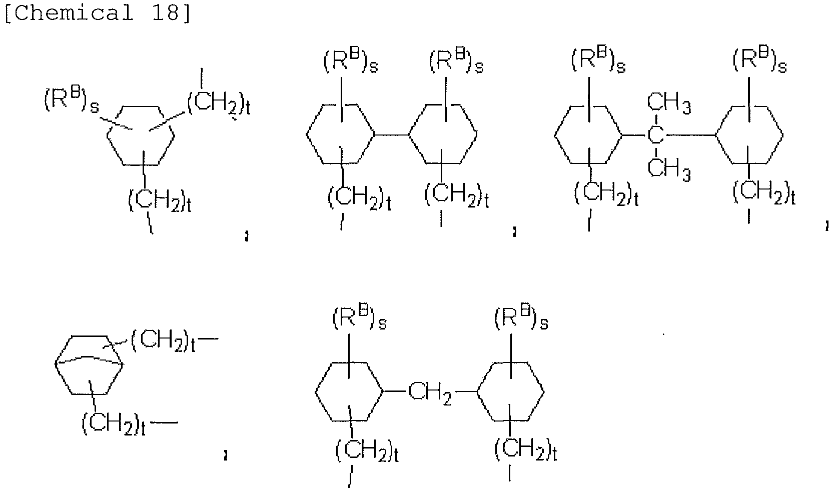 wherein R^{B} is an alkyl group having 1 to 4 carbon atoms or an alkoxy group having 1 to 4 carbon atoms, s represents the number of the substituents R^{B} and is an integer of 0 to 3, and t represents the number of chains of methylene and is an integer of 0 or 1.

In the above formula, t=0 means that the bonding hand is present on the aliphatic ring.

Among the divalent groups having the aliphatic ring represented by the above general formulas, most preferred are those represented by the following formulas:

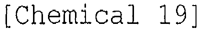

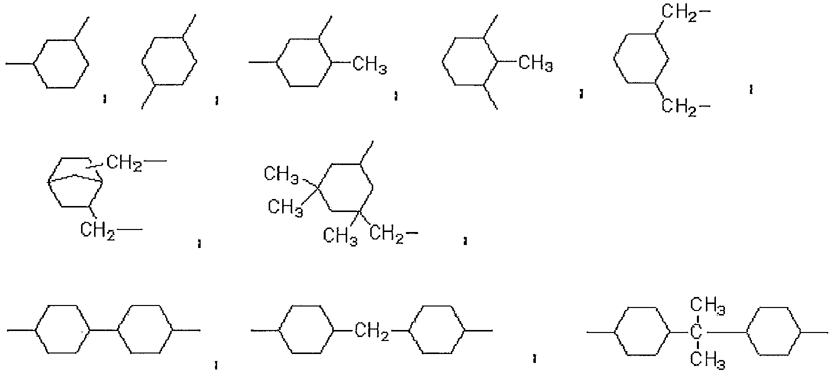

### Acyclic saturated hydrocarbon groups (R³, R⁶):

The acyclic saturated hydrocarbon groups having valencies of 2 to 6 may be in the form of either straight chain or branched but, preferably, have 1 to 15 carbon atoms and, specifically, 1 to 10 carbon atoms, and, specifically, are divalent groups (alkylene groups).

As the alkylene groups, there can be exemplified methylene group, ethylene group, trimethylene group, tetramethylene group, pentamethylene group and hexamethylene group which are of the form of a straight chain, as well as propylene group, isobutylene group and trimethylhexamethylene group which are of the branched form.

### Oxygen-containing hydrocarbon groups (R³, R⁶):

The oxygen-containing hydrocarbon groups of a structure in which the hydrocarbon groups coupled together through an oxygen-containing bridging group (-CO-, -COO-, - O-) are, preferably, the divalent groups such as the ones in which the two groups selected from alkylene group, cycloalkylene group and arylene group are bonded together through an oxygen-containing bridging group provided the total number of the carbon atoms is 1 to 30.

Concretely, the divalent groups represented by the following formula are preferred:

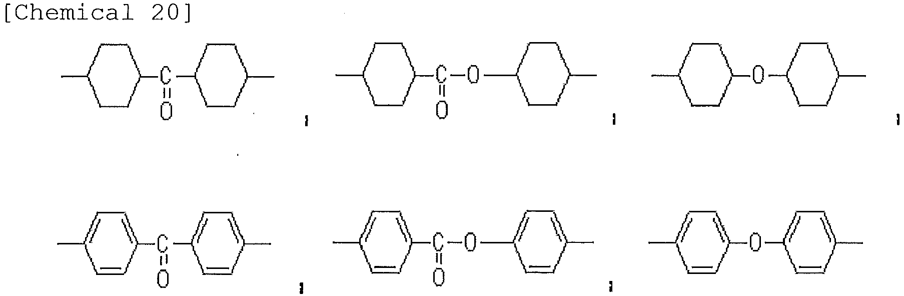

Described below are specifically preferred examples of the group R³ in the above general formula (1) and of the group R⁶ in the general formula (2).
Preferred groups R³ and R⁶;
methylene group, ethylene group, n-propylene group, isopropylene group, n-butylene group, n-pentylene group, n-hexylene group, cyclohexylene group, 2,2,4-trimethylhexylene group, phenylene group, naphthylene group, and groups represented by the following formulas:

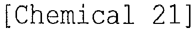

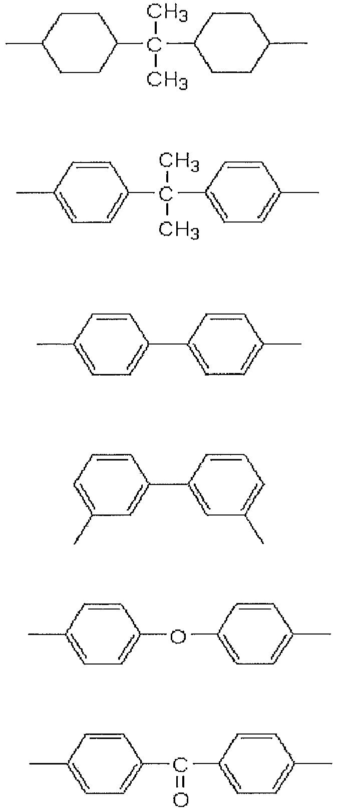

The (meth)acrylic - amide polymerizable monomers (A1-1) and (A1-2) represented by the above general formulas (1) and (2) may each be used in one kind alone or may be used in a mixture of two or more kinds. Described below are preferred examples.

Preferred examples of the (meth)acrylic - amide polymerizable monomer (A1-1):

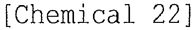

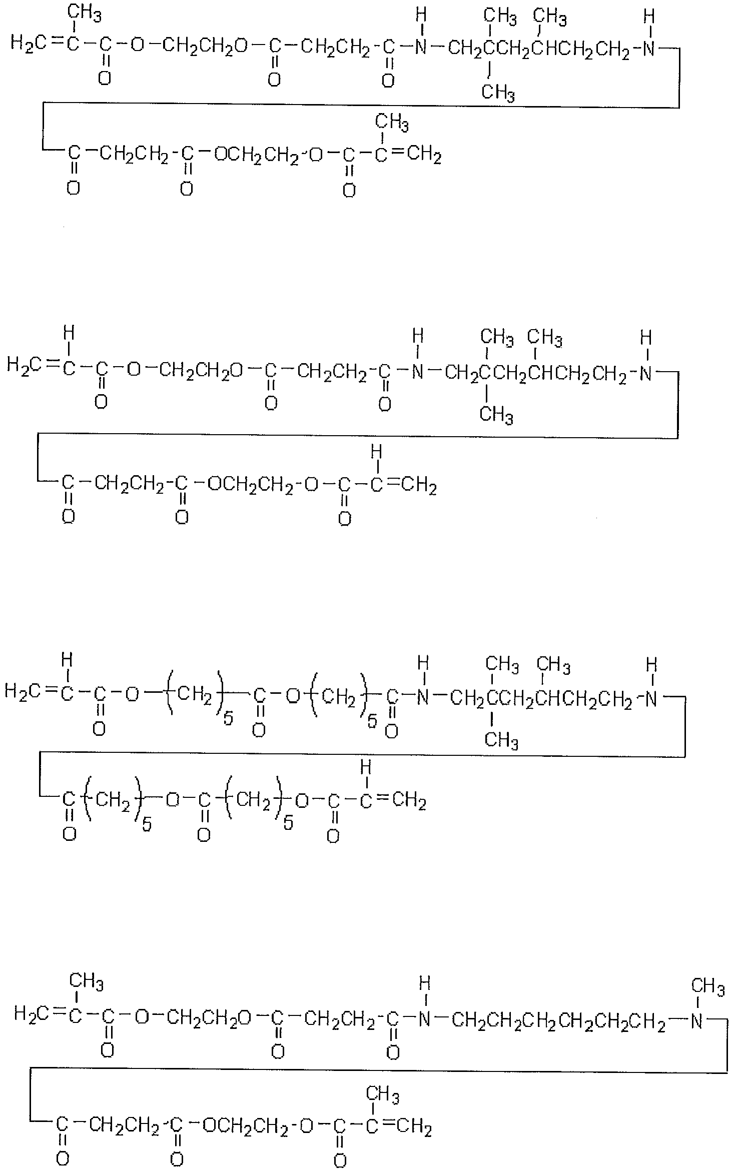

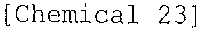

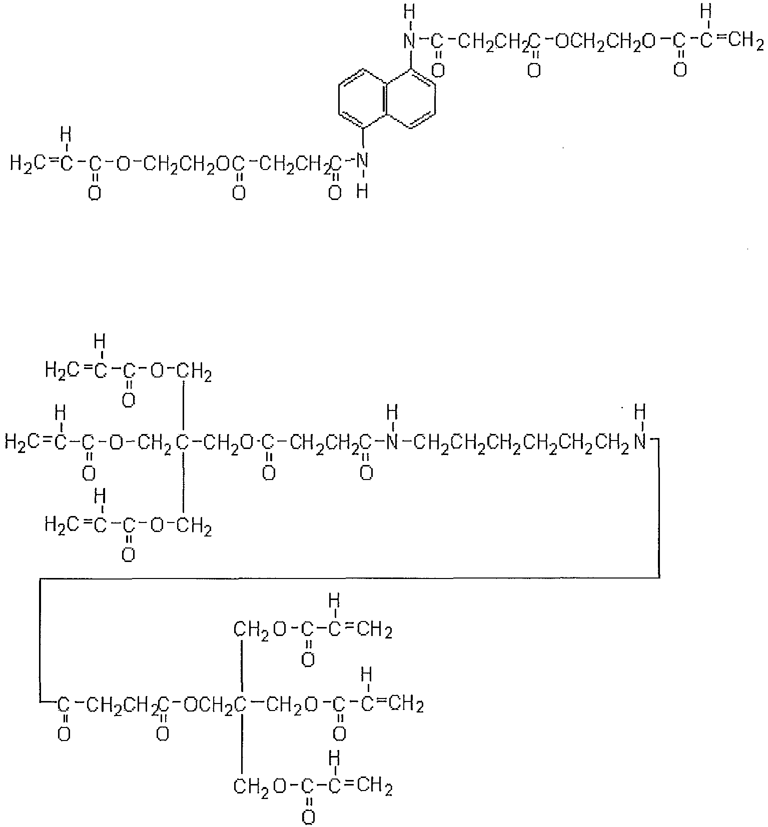

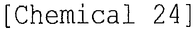

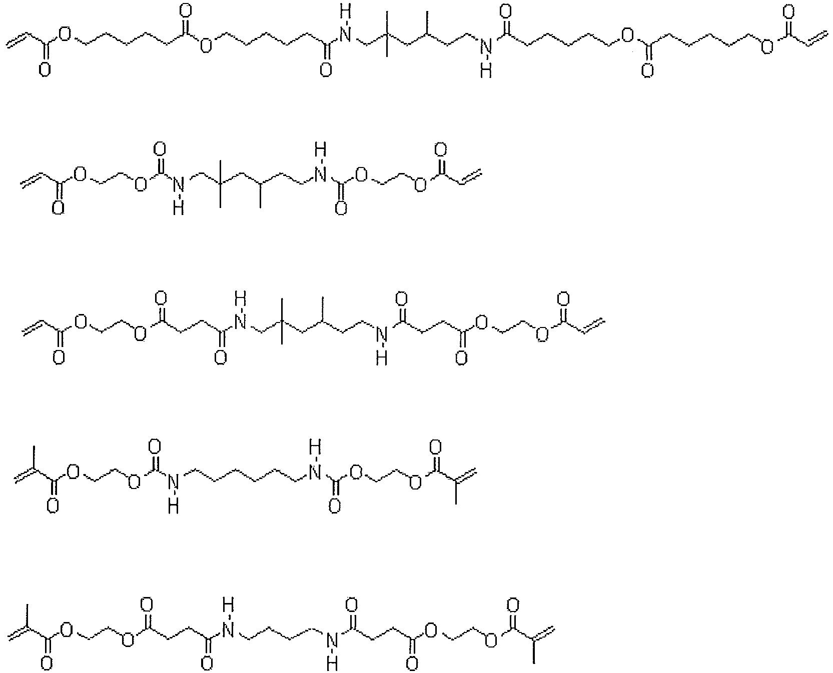

Preferred examples of the (meth)acrylic - amide polymerizable monomer (A1-2):

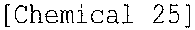

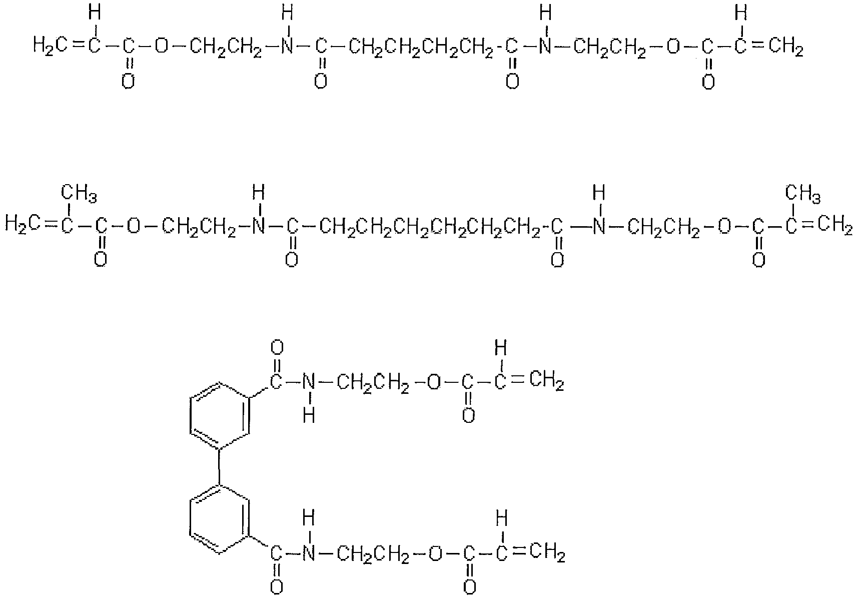

The (meth)acrylic - amide polymerizable monomer (A1-1) represented by the above general formula (1) is a novel compound as far as the present inventors know, and its production method will be described later in the last part of this specification.

The (meth)acrylic - amide polymerizable monomer (A1-2) represented by the general formula (2) is a known compound, and can be obtained according to, for example, a method described in JP-A-2000-204069 by transforming a compound having at least two carboxyl groups into an acid halide thereof, followed by the reaction with an aziridine compound to form an amide compound and, thereafter, reacting it with a compound having a (meth)acrylic group and a carboxylic acid.

The (meth)acrylic - amide polymerizable monomer is, usually, used in an amount of 10 to 80% by mass and, specifically, 20 to 50% by mass of the radically polymerizable monomer component (A) though the amount may vary depending on the kind thereof.

### 2. Non-amide polymerizable monomer (A2):

In the present invention, the non-amide polymerizable monomer (A2) used in combination with the (meth)acrylic - amide polymerizable monomer (A1) is the one that has at least three radically polymerizable groups but has no amide group. That is, the polymerizable monomer (A2) works as a crosslinking component. Use of this component makes it possible to improve the strength of the obtained cured body. For instance, if the polymerizable monomer having less than three radically polymerizable groups is used, then the cured body having a large strength cannot be obtained.

On the other hand, a photochromic compound is made present in the crosslinked spaces formed by the polymerization of the polymerizable monomer (A2). In the spaces, the photochromic compound undergoes the reversible reaction upon the irradiation with light, and color develops and fades repetitively. That is, as described earlier, if the cured body is formed by using the non-amide polymerizable monomer (A2) only, the crosslinked spaces become so rigid that a large mechanical strength can be obtained imposing, however, limitation on the freedom of the photohromic compound and impairing photochromic properties. The present invention, therefore, uses the above-mentioned (meth)acrylic - amide polymerizable monomer (A1) in combination.

In the present invention, there is no limitation on the structure of the non-amide polymerizable monomer (A2) so far as it has at least three radically polymerizable groups. From the standpoint of forming a densely crosslinked structure and obtaining a large mechanical strength, however, there is preferably used the following (meth)acrylic non-amide polymerizable monomer (A2-1) or the silicone type non-amide polymerizable monomer (A2-2) either in one kind or in a combination of two or more kinds.

### ((meth)acrylic non-amide polymerizable monomer (A2-1))

The non-amide polymerizable monomer (A2-1) of this type is represented by the following general formula (3): 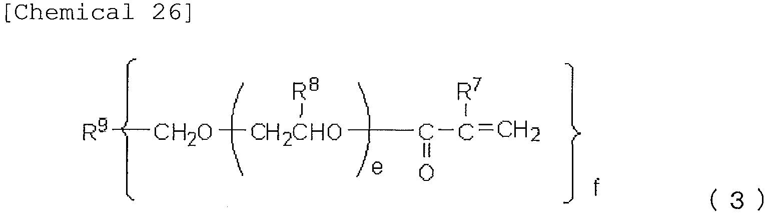 wherein,
R⁷ and R⁸ are hydrogen atoms or methyl groups,
R⁹ is an organic group having a valency of 3 to 6,
e is an integer of 0 to 3, and
f is an integer of 3 to 6.

In the general formula (3), examples of the organic group R⁹ having a valency of 3 to 6 are those represented by the following formula:

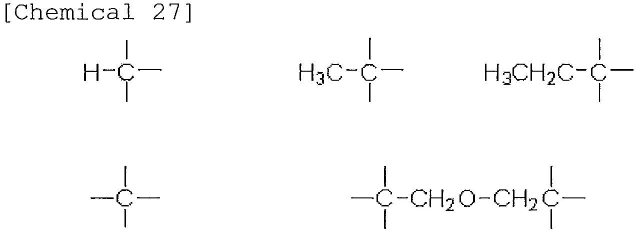

Among them, preferred are the following groups:

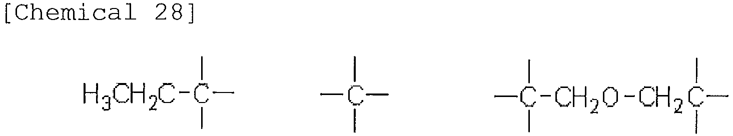

In the general formula (3), further, e is desirably an integer of 0 to 1, and f is desirably 3 or 4.

Described below are concrete examples of the non-amide polymerizable monomer (A2-1) represented by the general formula (3):
Trimethylolpropane trimethacrylate,
Trimethylolpropane triacrylate,
Tetramethylolmethane trimethacrylate,
Tetramethylolmethane triacrylate
Tetramethylolmethane tetramethacrylate,
Tetramethylolmethane tetraacrylate,
Trimethylolpropanetriethylene glycol trimethacrylate,
Trimethylolpropanetriethylene glycol triacrylate,
Ditrimethylolpropane tetramethacrylate,
Ditrimethylolpropane tetraacrylate,
Dipentaerythritol hexamethacrylate,
Dipentaerythritol hexaacrylate,
Polyester oligomer having 4 (meth)acrylic groups, and
Polyester oligomer having 6 (meth)acrylic groups.

Among the above non-amide polymerizable monomers (A2-1), those having a methacrylic group is preferred, and the trimethylolpropane trimethacrylate is, specifically, preferred from the standpoint of obtaining a specifically high color density and a fading rate.

### (Silicone non-amide polymerizable monomer (A2-2))

The non-amide polymerizable monomer of this type has an average composition represented by the following unit formula (4) :

R¹⁰SiO_{3/2} (4)

wherein,
R¹⁰ is a group bonded to a silicon atom, and is hydrogen atom, alkyl group, cycloalkyl group, alkoxy group, halogenated alkyl group, phenyl group, halogen-substituted phenyl group, hydroxyl group or an organic group containing a radically polymerizable group,
and in a molecule thereof, at least three groups R¹⁰ are organic groups containing a radically polymerizable group, and has a polymerization degree of 6 to 100.

Among the non-amide polymerizable monomers (A2), the silicone non-amide polymerizable monomer (A2-1) is most preferred.

In the silicone non-amide polymerizable monomer (often called silsesquioxane compound), examples of the organic group R¹⁰ containing radically polymerizable group are acrylic organic groups such as (meth)acrylic group, (meth)acryloxypropyl group, and (3-(meth)acryloxypropyl)dimethylsiloxy group; allyl organic groups such as allyl group, allylpropyl group and allylpropyldimethylsiloxy group; vinyl organic groups such as vinyl group, vinylpropyl group and vinyldimethylsiloxy group; cyclohexenyl organic groups such as (4-cyclohexenyl)ethyldimethylsiloxy group and the like; norbornenyl organic groups such as norbornenylethyl group and norbornenylethyl dimethylsiloxy group; and maleimide organic groups such as N-maleimidepropyl group and the like. Among them, specifically desired are the organic groups having a (meth)acrylic group and the organic groups having a vinyl group since they are capable of providing a large film strength yet exhibiting excellent photochromic properties.

As the alkyl group represented by R¹⁰, further, there can be exemplified alkyl groups having 1 to 10 carbon atoms, such as methyl group, ethyl group, n-propyl group, isopropyl group, n-butyl group, sec-butyl group, tert-butyl group, n-pentyl group, n-hexyl group, n-octyl and isooctyl group.

As the cycloalkyl group represented by R¹⁰, there can be exemplified cycloalkyl groups having 3 to 8 carbon atoms, such as cyclopropyl group, cyclobutyl group, cyclooctyl group, cyclohexyl group, cycloheptyl group and cyclooctyl group.

As the alkoxy groups represented by R¹⁰, there can be exemplified those having 1 to 6 carbon atoms, such as methoxy group, ethoxy group, n-propoxy group, isopropoxy group, n-butoxy group, sec-butoxy group and tert-butoxy group.

As the halogenated alkyl group represented R¹⁰, there can be exemplified those having 1 to 6 carbon atoms, such as trifluoromethyl group, pentafluoroethyl group, chloromethyl group, 2-chloroethyl group and bromomethyl group.

As the halogen-substituted phenyl group represented by R¹⁰, there can be exemplified 4-chlorophenyl group and 4-bromophenyl group.

The polymerization degree (corresponds to the number of silicon atoms to which oxygen atoms are bonded) of the silsesquioxane compound (A2-2) having an average composition of the formula (4), is 6 to 100 on the average but is, preferably, 8 to 50 and, specifically, 8 to 30 from the standpoint of photochromic properties and mechanical strength of the photochromic cured body (strength of the photochromic coating).

It is, further, desired that the number of the organic groups having radically polymerizable group possessed by the silsesquioxane compound (A2-2) is 3 to 50 and, specifically, 6 to 30 in one molecule thereof from the standpoint of photochromic properties and mechanical strength of the photochromic cured body. Further, the ratio of the organic groups having radically polymerizable group occupying the groups R¹⁰ bonded to the silicon atoms is, preferably, 10 to 100% and, specifically, 50 to 100%.

The silsesquioxane compound (A2-2) can assume a variety of structures such as of cage, ladder or random structure, and any one of them can be used for the present invention without limitation.

As the silsesquioxane compound (A2-2) that is preferred in the invention, there can be exemplified the one of the ladder structure represented by the following formula (5) and the one of the cage structure represented by the following formula (6).

### Silsesquioxane compound (A2-2) of the ladder structure:

A silsesquioxane compound represented by the following formula (6), 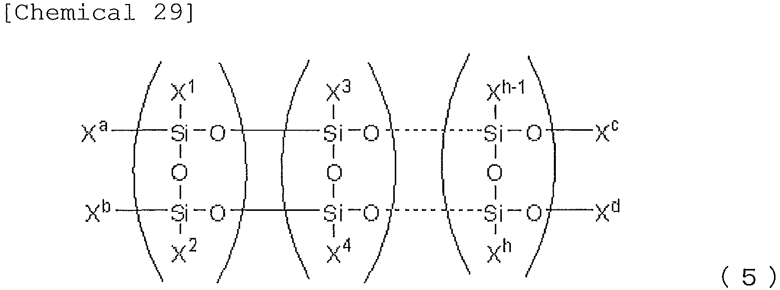 wherein,
X¹ to X^{h} may be the same or different, and are the groups exemplified for the group R¹⁰ in the formula (4), and at least three of X¹ to X^{h} are organic groups containing a radically polymerizable group,
terminal groups X^{a} and X^{b} are hydroxyl groups or alkoxy groups,
terminal groups X^{c} and X^{d} are hydrogen atoms or alkoxy groups, and
h is a polymerization degree and is an integer of 6 to 100.

### Silsesquioxane compound (A2-2) of the cage structure:

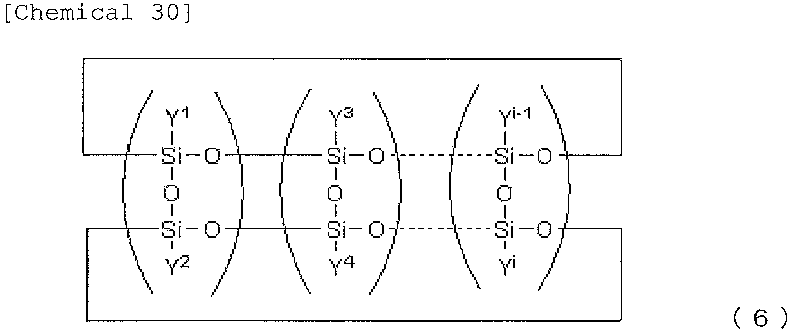

wherein,
Y¹ to Y¹ may be the same or different, and are the groups exemplified for the group R¹⁰ in the formula (4), and at least three of Y¹ to Yⁱ are organic groups containing a radically polymerizable group, and
i is a polymerization degree and is an integer of 6 to 12.

In the silsesquioxane compound (A2-2) of the cage structure, the number of the radically polymerizable groups in the substituents Y¹ to Yⁱ is, preferably, 3 to 12 and, specifically, 6 to 10. Further, the ratio of the radically polymerizable groups occupying the substituents Y¹ to Yⁱ is, preferably, 10 to 100% and, specifically, 50 to 100%.

Among the silsesquioxane compounds (A2-2) of the cage structure, specifically desired are those compounds having the following structures:
T8 structure (the one of a hexahedral structure in which i is 8 and having 8 silicon atoms at corners in the above formula (6));
T10 structure (the one of a heptahedral structure in which i is 10 and having 10 silicon atoms at corners in the above formula (6)); and
T12 structure (the one of an octahedral structure in which i is 12 and having 12 silicon atoms at corners in the above formula (6)).

These compounds are of the cage structure and it is considered the radically polymerizable groups (e.g., (meth)acryic groups) are fixed more rigidly and three-dimensionally. Therefore, the obtained cured body secures free space yet maintaining high hardness, which is advantageous in expressing excellent photochromic properties such as high color density and high fading rate.

The above silsesquioxane compound (A2-2) can be produced by a known method, or may be the one that is placed on the market.

For example, the following products are available in the market containing cage-structured silsesquioxane compounds.

### AC-SQ TA-100 produced by Toa Gosei Co.:

A mixture of a polysiloxane compound containing polyacryloxypropylpolyorganosiloxane (cage-structured T8). MAC-SQ TM-100 produced by Toa Gosei Co.:

A mixture of a polysiloxane compound containing polymethacryloxypropylpolyorganosiloxane (cage-structured T8).

### Q-8 produced by Toa Gosei Co.:

Octa[(3-methacryloxypropyl)dimethylsiloxy] silsesquioxane

### Q-6 produced by Toa Gosei Co.:

Octa[2-(vinyl)dimethylsiloxy] silsesquioxane MA0735 produced by HYBRID Plastics Co.

Polymethacryloxypropylpolyorganosiloxane compound (a mixture of cage-structured T8, T10 and T12) OL1660 produced by HYBRID Plastics Co.

Octavinylpolyorganosiloxane (cage-structured T8)

It is desired that the above non-amide polymerizable monomer (A2) is, usually, used in an amount of, preferably, 10 to 500 parts by mass, specifically, 20 to 300 parts by mass and, most preferably, 30 to 100 parts by mass per 100 parts by mass of the above-mentioned (meth)acrylic - amide polymerizable monomer (A1). Upon satisfying the above range, the obtained photochromic cured body exhibits excellent photochromic properties and large mechanical strength.

### 3. Other non-amide polymerizable monomers (A3):

In the present invention, it is also allowable to use, as the radically polymerizable monomer component (A), a non-amide polymerizble monomer (A3) other than the above (meth)acrylic - amide polymerizable monomer (A1) and the non-amide polymerizable monomer (A2).

The non-amide polymerizable monomer (A3) is used for adjusting properties of the curable composition or of the cured body, and has one or two radically polymerizable groups but has no amide group.

As the non-amide polymerizable monomer (A3), there can be preferably used the following bifunctional (meth)acrylic polymerizable monomer (A3-1), monofunctional (meth)acrylic polymerizable monomer (A3-2) and monofunctional (meth)acrylsilane polymerizable monomer (A3-3) either in one kind or in a mixture of two or more kinds.

### (Bifunctional (meth)acrylic polymerizable monomer (A3-1)):

This radically polymerizable monomer is represented by the following formula (7), 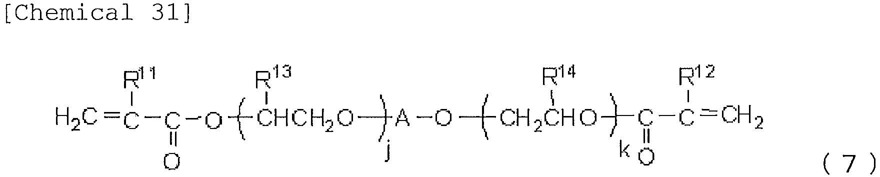 wherein,
j is 0 to 30, k is 0 to 30,
an average value of j+k is 2 to 30 and, preferably, 2 to 15,
R¹¹, R¹², R¹³ and R¹⁴ are a hydrogen atom or a methyl group, respectively,
A is an alkylene group; an unsubstituted phenylene group; a substituted phenylene group having, as a substituent, a halogen atom or an alkyl group having 1 to 4 carbon atoms; or a group represented by any one of the following formulas provided the number of carbon atoms is 1 to 20: 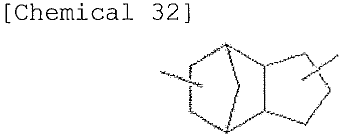 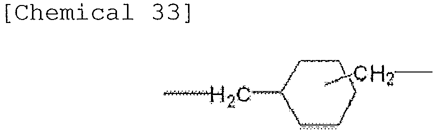 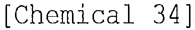 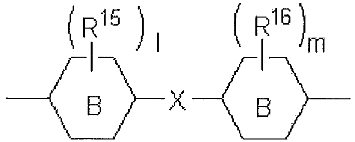 (wherein,
R¹⁵ and R¹⁶ are, respectively, an alkyl group having 1 to 4 carbon atoms or a halogen atom, 1 and m are, respectively, an integer of 0 to 4, the ring B in the formula is a benzene ring or a cyclohexane ring,
if the ring B is a benzene ring, X is -O-, -S-, -S(O₂)-, -CO-, -CH₂-, -CH=CH-, -C(CH₃)₂-, -C(CH₃) (C₆H₅)-, or a group represented by the following formula, 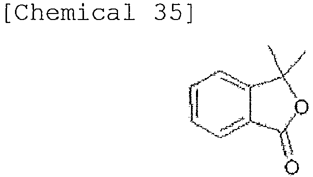 wherein,
if the ring B is a cyclohexane ring, X is -O-, -S-, -CH₂- or -C(CH₃)₂-).

Use of the bifunctional (meth)acrylic polymerizable monomer (A3-1) is advantageous for it makes it easy to handle the photochromic curable composition by lowering the viscosity thereof and secures photochromic properties and, specifically, a high color density and a high fading rate of the obtained cured body.

In the formula (7), the alkylene group denoted by A may be ethylene group, propylene group, butylene group or nonylene group.

Further, the substituted phenylene group denoted by A may be dimethylphenylene group, tetramethylphenylene group, dibromophenylene group or tetrabromophenylene group.

Described below are concrete examples of the above bifunctional (meth)acrylic polymerizable monomer (A3-1):
Ethylene glycol dimethacrylate,
Diethylene glycol dimethacrylate,
Triethylene glycol dimethacrylate,
Tetraethylene glycol dimethacrylate,
Polyethylene glycol dimethacrylate (average length of ethylene glycol chain, 9; average molecular weight, 536),
Polyethylene glycol dimethacrylate (average length of ethylene glycol chain, 14; average molecular weight, 736),
Polyethylene glycol dimethacrylate (average length of ethylene glycol chain, 23; average molecular weight, 1136),
Tripropylene glycol dimethacrylate,
Tetrapropylene glycol dimethacrylate,
Polypropylene glycol dimethacrylate (average
length of propylene glycol chain, 9; average molecular weight, 662),
Ethylene glycol diacrylate,
Diethylene glycol diacrylate,
Triethylene glycol diacrylate,
Tetraethylene glycol diacrylate,
Polyethylene glycol diacrylate (average length
of ethylene glycol chain, 9; average molecular weight, 508),
Polyethylene glycol diacrylate (average length
of ethylene glycol chain, 14; average molecular weight, 708),
Dipropylene glycol diacrylate,
Tripropylene glycol diacrylate,
Tetrapropylene glycol diacrylate,
Polypropylene glycol diacrylate (average
length of propylene glycol chain, 7; average molecular weight, 536),
Polypropylene glycol diacrylate (average
length of propylene glycol chain, 12; average molecular weight, 808),
1,3-Butanediol dimethacrylate,
1,6-Hexanediol dimethacrylate,
1,9-Nonanediol dimethacrylate,
1,10-Decanediol dimethacrylate,
Neopentyl glycol dimethacrylate,
2,2-Bis[4-methacryloxy(polyethoxy)phenyl] propane (average value of j+k, 2.3),
2,2-Bis[4-methacryloxy(polyethoxy)phenyl] propane (average value of a+b, 2.6),
2,2-Bis[4-methacryloxy(polyethoxy)phenyl] propane (average value of j+k, 4),
2,2-Bis[4-methacryloxy(polyethoxy)phenyl] propane (average value of j+k, 10),
2,2-Bis[4-methacryloxy(polyethoxy)phenyl] propane (average value of j+k, 20),
2,2-Bis[4-methacryloxy(polyethoxy)phenyl] propane (average value of j+k, 30),
Tricyclodecanedimethanol dimethacrylate, 1,6-Hexanediol diacrylate,
1,9-Nonanediol diacrylate,
1,10-Decanediol diacrylate,
Neopentyl glycol diacrylate,
Tricyclodecanedimethanol diacrylate,
Dioxane glycol diacrylate,
Ethoxylated cyclohexanedimethanol diacrylate (average value of j+k, 4),
2,2-Bis[4-acryloxy(polyethoxy)phenyl] propane (average value of j+k, 3),
2,2-Bis[4-methacryloxy(polyethoxy)phenyl] propane (average value of j+k, 4), and
2,2-Bis[4-methacryloxy(polyethoxy)phenyl] propane (average value of j+k, 10).

Of the monomers exemplified above, preferred are those of which A in the above formula (7) is ethylene group, propylene group or the one having a molecular skeleton represented by the following formula, 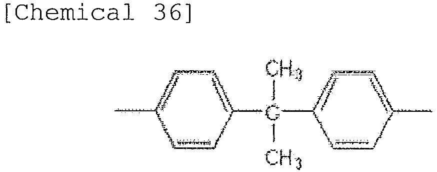 from the standpoint of obtaining a specifically high color density.

### (Monofunctional (meth)acrylic polymerizable monomer (A3-2))

This monomer (A3-2) is represented by the following formula (8), 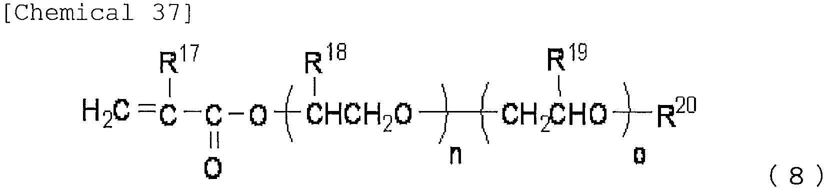 wherein,
R¹⁷, R¹⁸ and R¹⁹ are hydrogen atoms or methyl groups,
R²⁰ is a hydrogen atom, an alkyl group having 1 to 20 carbon atoms, a cycloalkyl group having 6 to 20 carbon atoms, a phenyl group which may be substituted with an alkyl group having 1 to 20 carbon atoms, a naphthyl group which may be substituted with an alkyl group having 1 to 20 carbon atoms, or a glycidyl group,
n is 0 to 25, o is 0 to 25, and
an average value of n+o is 0 to 25 and, specifically, 0 to 15.

The monofunctional (meth)acrylic polymerizable monomer (A3-2), too, is obtained, usually, in the form of a mixture of molecules of different molecular weights like the above-mentioned monomer (A3-1). Therefore, the values of n and o are on the average.

Described below are concrete examples of the monofunctional (meth)acrylic polymerizable monomer (A3-2):
Methoxydiethylene glycol methacrylate,
Methoxytetraethylene glycol methacrylate,
Isostearyl methacrylate,
Isobornyl methacrylate,
Phenoxyethylene glycol methacrylate,
Phenoxyethyl acrylate,
Phenoxydiethylene glycol acrylate,
Naphthoxyethylene glycol acrylate,
Isostearyl acrylate,
Isobornyl acrylate,
Glycidyl methacrylate,
Methoxypolyethylene glycol methacrylate (average length of ethylene glycol chain, 9; average molecular weight, 468),
Methoxypolyethylene glycol methacrylate (average length of ethylene glycol chain, 23; average molecular weight, 1068), and
Phenoxypolyethylene glycol acrylate (average length of ethylene glycol chain, 6; average molecular weight, 412).

### (Monofunctional (meth)acrylsilane polymerizable monomer (A3-3))

This monomer (A3-3) is represented by the following formula (9), 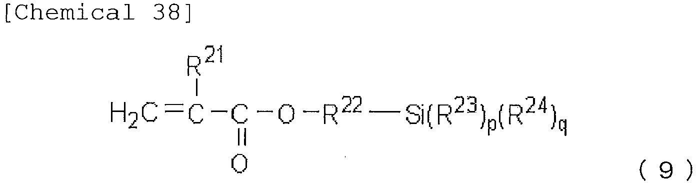 wherein,
R²¹ is a hydrogen atom or a methyl group,
R²² is an alkylene group having 1 to 10 carbon atoms,
R²³ is an alkoxy group having 1 to 6 carbon atoms,
R²⁴ is an alkyl group having 1 to 6 carbon atoms, and
p is an integer of 1 to 3, and q is an integer of 0 to 2, but p+q being 3.

As the alkylene group represented by the above group R²², there can be exemplified ethylene group, propylene group and butylene group.

As the alkoxy group represented by the group R²³, further, there can be exemplified methoxy group, ethoxy group and propoxy group.

Further, as the alkyl group represented by the group R²⁴, there can be exemplified methyl group, ethyl group and propyl group.

Described below are preferred examples of the monofunctional (meth)acrylsilane polymerizable monomer (A3-3) :
γ-Methacryloyloxypropyltrimethoxysilane,
γ-Methacryloyloxypropyltriethoxysilane, and
γ-Methacryloyloxypropylmethyldimethoxysilane.

As the other non-amide polymerizable monomers (A3) suitably used in the invention, further, there can be used urethane(meth)acrylates and vinyl polymerizable monomers (e.g., styrene, α-methylstyrene, α-methylstyrene dimer, divinylbenzene, etc.) in addition to those described above.

The above-mentioned other amide polymerizable monomer (A3) can be used in a suitable amount provided the (meth)acrylic - amide polymerizable monomer (A1) and the non-amide polymerizable monomer (A2) are contained in the above-mentioned amounts. For example, the other non-amide polymerizable monomer (A3) can be used in an amount of not more than 87% by mass provided the radically polymerizable monomer component (A) contains the (meth)acrylic - amide polymerizable monomer (A1) in an amount of 10 to 80% by mass and contains the non-amide polymerizable monomer (A2) in an amount of 3 to 50% by mass.

In the present invention, it is particularly desired that the (meth)acrylic - amide polymerizable monomer (A1) and the non-amide polymerizable monomer (A2) are contained in amounts mentioned above and, further, that the polymerizable monomers (A1) to (A3) are contained at ratios described below from the standpoint of improving the mechanical strength of the photochromic cured body and the strength of the photochromic coating yet maintaining photochromic properties thereof.
(Meth)acrylic - amide polymerizable monomer(A1):
15 to 65% by mass and, specifically, 20 to 50% by mass;
Non-amide polymerizable monomer (A2): 5 to 45% by mass and, specifically, 7 to 40% by mass;
Other non-amide polymerizable monomer (A3): not more than 80% by mass and, specifically, 10 to 73% by mass.

Further, when the other non-amide polymerizable monomer (A3) is used, it is desired that 70 to 99% by mass of the monomer (A3) is the above-mentioned bifunctional (meth)acrylic polymerizable monomer (A3-1).

### <Photochromic compound (B)>

The photochromic curable composition of the present invention is blended with a photochromic compound (B) for imparting photochromic properties.

The photochromic compound can be represented by chromene compound, fulgimide compound, spirooxazine compound and spiropyran compound. The present invention can use any of these known photochromic compounds either in a single kind or in a combination of two or more kinds.

Typical examples of the fulgimide compound, spirooxazine compound, spiropyran compound and chromene compound have been described in, for example, JP-A-2-28154, JP-A-62-288830, WO94/22850 and WO96/14596.

As the chromene compounds, further, there have been known the ones having excellent photochromic properties in addition to those described in the above patent documents, and such chromene compounds, too, can be preferably used. The chromene compounds having excellent photochromic properties have been closely described in the following patent documents together with their structures.
JP-A-2001-031670; JP-A-2001-011067;
JP-A-2001-011066; JP-A-2000-344761;
JP-A-2000-327675; JP-A-2000-256347;
JP-A-2000-229976; JP-A-2000-229975;
JP-A-2000-229974; JP-A-2000-229973;
JP-A-2000-229972; JP-A-2000-219678;
JP-A-2000-219686; JP-A-11-322739;
JP-A-11-286484; JP-A-11-279171;
JP-A-09-218301; JP-A-09-124645;
JP-A-08-295690; JP-A-08-176139;
JP-A-08-157467; USP5645767;
USP5658501; USP5961892;
USP6296785;
JP4424981; JP4424962;
WO2009/136668; WO2008/023828;
JP4369754; JP4301621;
JP4256985; WO2007/086532;
JP-A-2009-120536; JP-A-2009-67754;
JP-A-2009-67680; JP-A-2009-57300;
JP4195615; JP4158881;
JP4157245; JP4157239;
JP4157227; JP4118458;
JP2008-74832; JP3982770;
JP3801386; WO2005/028465;
WO2003/042203; JP-A-2005-289812;
JP-A-2005-289807; JP-A-2005-112772;
JP3522189; WO2002/090342;
JP3471073; JP2003-277381;
WO2001/060811; WO2000/171544;
WO2005/028465; WO2011/16582;
WO2011/034202; JPA-2011-050730.

Among these photochromic compounds, the present invention preferably uses a chromene compound having an indenonaphtho[2,1-f]naphtho[1,2-b] pyran skeleton from the standpoint of photochromic properties such as color density, initial color, light resistance and fading rate. Among these chromene compounds, further, the compounds having molecular weights of not smaller than 540 are preferred owing to their particularly excellent color density and fading rate. Described below are concrete examples.

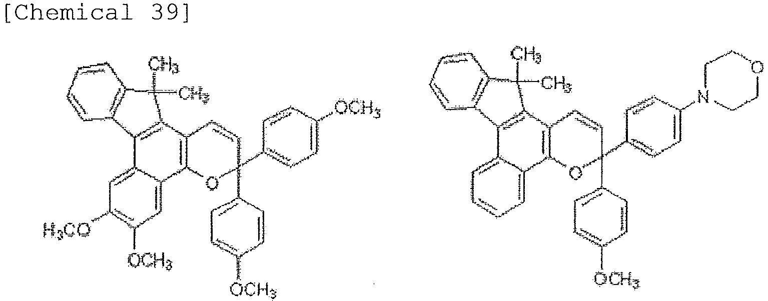

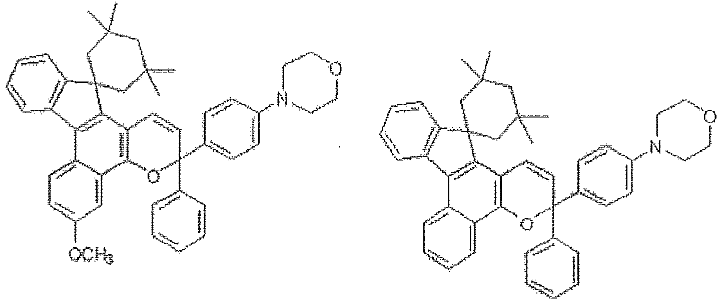

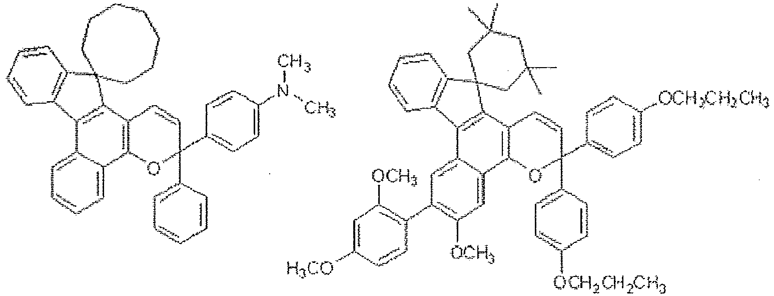

In the invention, the above-mentioned photochromic compound (B) is added in an amount of 0.01 to 20 parts by mass, specifically, 0.03 to 10 parts by mass and, most preferably, 0.05 to 5 parts by mass per 100 parts by mass of the above radically polymerizable monomer component. Namely, the photochromic compound (B) is homogeneously dissolved in the polymerizable monomer component (A), and there is obtained a cured body having a high color density without shade in the color.

### <Other blending agents (C)>

The photochromic curable composition of the invention can, as required, be mixed with various blending agents that have been known per se., such as radical polymerization initiator, ultraviolet-ray absorber, radical polymerization initiator, parting agent, infrared-ray absorber, ultraviolet stabilizer, antioxidant, anti-coloring agent, antistatic agent, fluorescent dye, dye, pigment, perfume and the like depending on the use in amounts in a range in which they do not impair the object of the invention.

The photochromic curable composition of the present invention uses, for example, a radical polymerization initiator to polymerize the above radically polymerizable monomer component (A). The radical polymerization initiator may have been added to the composition from the first time or may be added thereto at the time of preparing the photochromic cured body by the in-mass method or the coating method that will be described later.

Described below are representative thermal polymerization initiators and photo polymerization initiators.

As the thermal polymerization initiator, there can be exemplified:
Acyl peroxides such as:
Benzoyl peroxide,
p-Chlorobenzoyl peroxide,
Decanoyl peroxide,
Lauroyl peroxide,
Acetyl peroxide,
Peroxy esters such as:
t-Butylperoxy-2-ethyl hexanoate,
t-Butlperoxyneodecanoate,
Cumylperoxyneodecanoate,
t-Butylperoxybenzoate,
Percarbonates such as:
Diisopropylperoxydicarbonate,
Di-sec-butylperoxydicarbonate,
Azo compounds such as:
Azobisisobutylonitrile.

As the photo polymerization initiator, there can be exemplified:
Acetophenone compounds such as:
   1-Phenyl-2-hydroxy-2-methylpropane-1-one,
   1-Hydroxycyclohexylphenylketone,
   1-(4-Isopropylphenyl)-2-hydroxy-2-methylpropane-1-one,
α-Dicarbonyl compounds such as:
   1,2-Diphenylethane-dione,
   Methylphenyl glyoxylate,
Acylphosphinoxide compounds such as:
   2,6-Dimethylbenzoyldiphenyl phosphinoxide,
   2,4,6-Trimethylbenzoyldiphenyl phosphinoxide,
   Methyl 2,4,6-trimethylbenzoyldiphenylphosphinate,
   2,6-Dichlorobenzoyldiphenylphosphinoxide,
   2,6-Dimethoxybenzoyldiphenylphosphinoxide.

The above polymerization initiators may be used in one kind or in a mixture of two or more kinds. Further, the thermal polymerization initiator and the photo polymerization initiator may be used in combination. When the photo polymerization initiator is used, there can be used a known polymerization accelerator such as tertiary amine or the like.

The polymerization initiators are used in amounts in a range of 0.001 to 10 parts by mass and, specifically, 0.01 to 5 parts by mass per 100 parts by mass of the radically polymerizable monomer component (A).

The photochromic curable composition of the invention is desirably blended with an ultraviolet stabilizer to further improve light resistance of the photochromic compound.

As the ultraviolet ray stabilizer, there can be preferably used hindered amine photo stabilizer, hindered phenol antioxidant and sulfur type antioxidant. Other preferred examples include the following compounds and the commercially available products.
Bis(1,2,2,6,6-pentamethyl-4-piperidyl) sebacate;
ADEKA STAB LA-52, LA-57, LA-62, LA-63, LA-67, LA-77, LA-82, LA-87 produced by Asahi Denka Kogyo Co.;
2,6-Di-t-butyl-4-methylphenol;
2,6-Ethylenebis(oxyethylene)bis[3-(5-t-butyl-4-hydroxy-m-tolyl) propionate];
IRGANOX 1010, 1035, 1075, 1098, 1135, 1141, 1222, 1330, 1425, 1520, 259, 3114, 3790, 5057, 565 produced by Chiba Specialty Chemicals Co.

The ultraviolet ray stabilizers are, usually, used in amounts of about 0.001 to 10 parts by mass and, specifically, about 0.01 to 1 part by mass per 100 parts by mass of the radically polymerizable monomer component (A).

Further, when the color that develops is adjusted by using a plurality of kinds photochromic compounds, the hindered amine photo stabilizer, if it is used in excess amounts, may cause a difference in the light resistance among the photochromic compounds and may, therefore, cause a deviation in the developed color tone. Therefore, the hindered amine photo stabilizer is used in an amount of, preferably, 0.5 to 30 mols, more preferably, 1 to 20 mols and, more preferably, 2 to 15 mols per mol of the photochromic compound (the mol number of the hindered amine photo stabilizer is calculated with the hindered amine portion as one mol).

The photochromic curable composition of the present invention can be easily produced by mixing the above-mentioned various components thereto in a customary manner.

### <Preparation of the photochromic cured body>

The photochromic curable composition of the present invention is suitably blended with the above radical polymerization initiator and is radically polymerized according to a known method to obtain a desired photochromic cured body. The radical polymerization is carried out by being heated, by the irradiation with ultraviolet rays, α-rays, β-rays or γ-rays, or by both of them.

In the present invention, in particular, it is desired that the photochromic cured body is formed by the in-mass method or the coating method.

### 1. In-mass method.

The in-mass method employs a mold polymerization according to which the photochromic curable composition of the invention mixed with the radical polymerization initiator is injected into between the molds that are held by the elastomer gaskets or the spacers, is oxidized in the air furnace and is taken out. Thus, there is obtained a photochromic formed body imparted with photochromic properties and having a predetermined shape (e.g., lens shape).

Among the polymerization conditions under which the mold polymerization is carried out, the temperature, in particular, affects the properties of the obtained photochromic cured body. The temperature condition cannot be exclusively defined since it is subject to be affected by the kind and amount of the radical polymerization initiator and by the kinds of the monomers. Usually, however, the so-called tapered polymerization is desired by slowly elevating the temperature and effecting the curing at a high temperature at a moment when the polymerization is completed. Like the temperature, the polymerization time, too, varies depending on various factors. It is, therefore, desired that an optimum time is determined in advance depending upon these conditions. The conditions are, desirably, so selected that the polymerization completes in 2 to 24 hours.

### 2. Coating method.

According to the coating method, the photochromic curable composition of the present invention is used as a photochromic coating agent, is applied onto the surface of a predetermined base material, and is polymerized and cured to form a photochromic coating (photochromic cured body) on the surface of the base material.

There is no specific limitation on the base material that is to be coated, and examples thereof are, generally, optical base materials that have been widely known, such as spectacle lenses, windowpanes of houses and automobiles.

As the spectacle lenses, there have been known plastic type spectacle lenses such as of (meth)acrylic resin, polycarbonate resin, acrylic resin, thiourethane resin, urethane resin and thioepoxy resin, as well as glass type spectacle lenses.

The photochromic curable composition of the present invention can be used as a coating agent for the spectacle lenses of either type without any particular limitation, but can be more preferably used as a coating agent for the plastic type spectacle lenses and, further specifically, for the spectacle lenses of (meth)acrylic resin, polycarbonate resin, acrylic resin, thiourethane resin, urethane resin and thioepoxy resin.

The base material is coated with the photochromic coating agent by spin coating, spray coating, dip coating or dip-spin coating depending on the form of the base material.

Prior to applying the photochromic coating, it is desired that the base material is pretreated to improve wettability and close adhesion of the curable composition to the base material.

As the pretreatment, there can be exemplified chemical treatment using a basic aqueous solution or an acidic aqueous solution, polish treatment by using a polishing agent, a plasma treatment by using an atmospheric plasma and low-pressure plasma, corona discharge treatment or UV ozone treatment. Not being specifically limited to these methods, any known method may be used; i.e., these methods may also be used in combination to improve close adhesion to the optical base material.

The photochromic coating agent after applied is cured by the irradiation with light or by heating. Preferably, however, the photochromic coating agent is cured by the irradiation with light and is, further, completely polymerized by heating.

The thickness of the thus obtained photochromic coating (photochromic cured body) is not specifically limited but is maintained to be relatively large from the standpoint of obtaining a sufficiently large color density despite the concentration of the photochromic compound is low and of obtaining good repeat durability. However, if the thickness of the coating is too large, the initial yellow color becomes dense. Therefore, the thickness of the photochromic coating is about 10 to about 100 µm and, specifically, about 20 to about 50 µm.

Here, depending upon the use, the photochromic cured body obtained by the above in-mass method or the coating method is subjected to the following treatments.

Namely, the photochromic cured body can be subjected to the working and the secondary treatment, such as antireflection treatment or antistatic treatment using a dye like dispersion dye, using a hard coating comprising chiefly a silane coupling agent or a sol such as of silicon, zirconium, antimony, aluminum, tin or tungsten, or being deposited with a thin film of a metal oxide such as SiO₂, TiO₂ or ZrO₂, or being applied with an organic high molecular thin film.

### <Preparation of the (meth)acrylic - amide polymerizable monomer (A1-1)>

The (meth)acrylic - amide polymerizable monomer (A1-1) represented by the above general formula (1), so far as the present inventors know, is a novel compound which is produced by a method described below.

A carboxylic acid derivative of the following formula (1X) is used as the starting material. 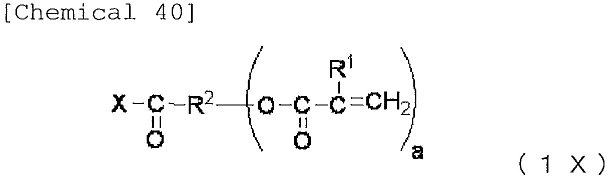 wherein,
R¹, R² and a are as defined in the above formula (1),
X is a hydroxyl group, a chlorine atom, a bromine atom, a group -N₃ or a group represented by the following formula,

   R³⁷-COO-
(wherein R³⁷ is an alkyl group or an aryl group).

The carboxylic acid derivative and an amine compound represented by the following formula (1Y),

R³-(NH₂)_{b} (1Y)

wherein R³ and b are as defined in the above formula (1),
are reacted in a mixed solvent of an organic solvent and water in the presence of a basic substance to produce an amide bond-containing monomer in a high yield forming little by-products.

The reaction of the carboxylic acid derivative with the amine compound proceeds as described below. In the following formula, b is the number as defined in the general formula (1).

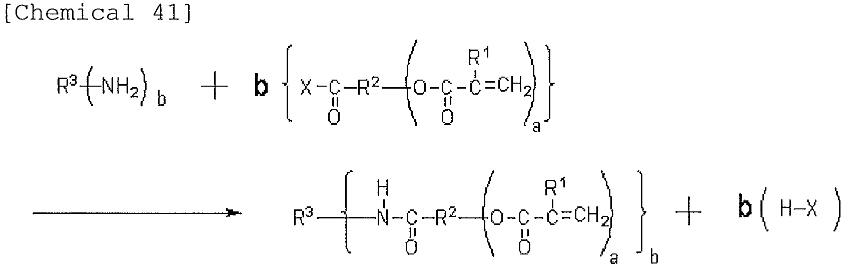

### Carboxylic acid derivatives.

In the carboxylic acid derivative used as the starting material, R¹, R² and a in the formula (1X) are as defined in the above formula (1), and are as described in the general formula (1). Here, however, X in the formula (1X) is a hydroxyl group, a chlorine atom, a bromine atom, a group -N₃, or a group represented by R³⁷-COO-.

The above group R³⁷ is an alkyl group or an aryl group.

The alkyl group is, preferably, an alkyl group having 1 to 8 carbon atoms and, specifically preferably, a methyl group or an ethyl group from the standpoint of easy availability of the starting material.

Further, the aryl group is, preferably, a phenyl group or a substituted phenyl group having a substituent. The substituent is, preferably, an electron-attracting group such as chloro group or nitro group. Preferred examples of the aryl group include 2-chlorophenyl group, 2,4-dichlorophenyl group, 2,6-dichlorophenyl group, 2,4,6-trichlorophenyl group and 4-nitrophenyl group.

The carboxylic acid derivative represented by the formula (1X) in which X is a chlorine atom, a bromine atom or a group of the formula R³⁷-COO-, can be easily synthesized, usually, from a carboxyl group-containing monomer in which X is a hydroxyl group.

For example, the one (acid halide) in which X is a chlorine atom or a bromine atom can be easily synthesized by reacting a corresponding carboxyl group-containing monomer with a halogenating agent such as thionyl chloride.

Further, the one in which X is represented by the formula R³⁷-COO- can be obtained in the form of an acid anhydride by reacting a corresponding carboxyl group-containing monomer with an acid chloride that contains R³⁷.

In the invention, the carboxylic acid derivative in which X is a chlorine atom can be most easily synthesized.

Here, the carboxylic acid derivative used as a starting material has a polymerizable group. Namely, the (meth)acrylic - amide polymerizable monomer (A1-1) of the general formula (1) can be synthesized by using a carboxylic acid derivative having a polymerizable group and offers such an advantage that it can be reacted under a mild condition yet suppressing the formation of by-products as compared with those synthesized by using, for example, an amine compound having a polymerizable group. Use of the carboxylic acid derivative having a polymerizable group is advantageous from the standpoint of availability of the starting material and synthesis, too.

### Amine compounds.

In the amine compound represented by the formula (1Y), i.g.,

R³-(NH₂)_{b} (1Y)

R³ and b are as defined in the above formula (1).

The amine compound may be used in an amount of (1/b) mol equivalent per mole of the carboxylic acid derivative. For example, if there is used a diamine compound of the formula (1Y) in which b is 2, then the amount of use thereof is, most desirably, 0.5 mol equivalent per the carboxylic acid derivative. If the amount thereof is smaller than the above, then the carboxylic acid derivative remains in excess amounts and if the amount thereof is larger than the above, then the intermediate product (amino product) may remain.

### Reaction solvents.

The reaction of the carboxylic acid derivative with the amine compound is carried out in a mixed solvent of an organic solvent and water.

There is no specific limitation on the organic solvent in the mixed solvent if it permits the starting material to dissolve therein and does not react with the starting material or the formed product.

Usually, there are used ketone solvents such as acetone, methyl ethyl ketone and methyl isobutyl ketone; ester solvents such as ethyl acetate, propyl acetate and butyl acetate; aromatic solvents such as benzene, toluene and xylene; ether solvents such as diethyl ether, butylmethyl ether, dipropyl ether, dibutyl ether, tetrahydrofuran, dioxane and dimethoxyethane; aliphatic solvents such as hexane, heptane, cyclohexane and decahydronaphthalene; and halogen solvents such as dichloromethane, chloroform and carbon tetrachloride.

Among them, preferred examples are ketone solvents, ether solvents, aromatic solvents and halogen solvents from the standpoint of highly dissolving and dispersing the carboxylic acid derivative therein. If operability in the after-treatment is taken into consideration, further preferred are ether solvents, aromatic solvents and halogen solvents. Concretely, there can be specifically preferably used diethyl ether, dimethoxyethane, tetrahydrofuran, toluene, dichloromethane and chloroform.

There is no specific limitation on the amount of the organic solvent that is used if it is enough for dissolving the starting material but the amount thereof is, preferably, in a range of 0.01 to 200 times (mass basis) as much as the weight of the carboxylic acid derivative. Upon satisfying this range, it is allowed to adjust the rate of reaction, to suppress the formation of by-products and to simplify the step of after-treatment. By taking into consideration the rate of reaction, suppressing the formation of by-products and simplifying the step of after-treatment, it is desired that the organic solvent is used in an amount of 0.1 to 100 times (mass basis) as much as the amount of the organic solvent.

There is no specific limitation on the water that is used as the reaction solvent if it is used in an amount sufficient for dissolving the basic material that will be described later. Water, however, is preferably used in an amount of 0.1 to 10 times and, specifically, 1 to 5 times as much as the amount of the organic solvent on the mass basis.

### Basic substances.

The reaction of the carboxylic acid derivative with the amine compound is carried out in the presence of a basic substance.

Described below are examples of the basic substance.

### Tertiary amines:

Triethylamine, diisopropylethylamine, triisopropylamine, tributylamine, N,N,N,N-tetramethylethylenediamine, etc..

### Nitrogen atom-containing aromatic heterocyclic compounds:

Pyridine, etc.

### Hydroxides of alkali metals:

Lithium hydroxide, sodium hydroxide, potassium hydroxide, etc.

### Hydrogen carbonates:

Sodium hydrogencarbonate, potassium hydrogencarbonate, etc.

### Carbonates:

Sodium carbonate, potassium carbonate, etc.

Among the above basic substances, preferred examples are hydroxides, hydrogen carbonates and carbonates from such a standpoint that they exhibit suitable degrees of basic property making it possible to effectively remove acids formed by condensation. Specifically preferred examples are sodium hydroxide, potassium hydroxide, sodium hydrogencarbonate, potassium hydrogencarbonate, sodium carbonate and potassium carbonate.

It is desired that the basic substance is used in an amount in a range of 0.5 to 10 mol equivalents and, specifically, 1 to 5 mol equivalents per mol equivalent of the carboxylic acid derivative. Upon satisfying the above range, acid formed by the reaction is more neutralized, formation of by-products is suppressed, and the yield and purity can be improved. When the compound containing an acrylic acid as the polymerizable group is used as the starting material, in particular, the Michel addition reaction occurs due to unsaturated bond of the acrylic group and the by-product easily forms. Therefore, the base is used in an amount of not less than 0.5 mol equivalents and, preferably, not less than one mol equivalent per mol equivalent of the carboxylic acid derivative.

### Reaction conditions.

The carboxylic acid derivative is reacted with the amine compound by adding the basic substance to the mixed solvent of the above organic solvent and water, adding the amine compound thereto and, further, adding the carboxylic acid derivative thereto. In this case, the carboxylic acid derivative is used in its form or in the form of a solution by dissolving it in an organic solvent.

The reaction temperature is, preferably, in a range of -80°C to 100°C and, specifically preferably, in a range of 0°C to 60°C. With the reaction temperature satisfying the above range, the reaction easily proceeds, formation of by-products is suppressed, and yield and purity are improved. The reaction time may be suitably determined by confirming the rate of consumption of the starting materials. Usually, however, the reaction time of 1 to 12 hours is sufficient.

The obtained (meth)acrylic - amide polymerizable monomer (A1-1) can be directly used after having removed the reaction solvent, base and formed salts therefrom, but is, preferably, used in a refined form. There is no specific limitation on the refining method, and the refining can be conducted by distillation, recrystallization or column chromatography.

### Identifying the (meh)acrylic - amide polymerizable monomer (A1-1).

The (meth)acrylic - amide polymerizable monomer (A1-1) obtained above is, usually, present as a viscous liquid which is colorless or faintly yellow at normal temperature and under normal pressure, and can be confirmed by the following means (a) to (c).
(a) Measurement of the proton nuclear magnetic resonance spectra (¹H-NMR) shows peaks based on the vinyl proton of the unsaturated bond near δ: 5.0 to 7.0 ppm, and peaks based on the protons of an alkyl group and an alkylene group near δ: 0.5 to 4.9 ppm. Further, the number of protons of the bonding groups can be learned from a relative comparison of the respective spectral intensities.
(b) The compositions of the corresponding products can be determined by the elemental analysis.
(c) The infrared absorption analysis (IR) reveals a peak based on the amide bond at 1650 cm⁻¹.

### EXAMPLES

The invention will be described below in further detail by way of Examples to which only, however, the invention is in no way limited. Described below are abbreviations and names of the compounds that are used.

### <(Meth)acrylic - amide polymerizable monomer (A1)>

In the following polymerizable monomers, the AM-03 (A1-2) only is represented by the general formula (2) and others (A1-1) are all represented by the general formula (1).
AM-01 (A1-1) : 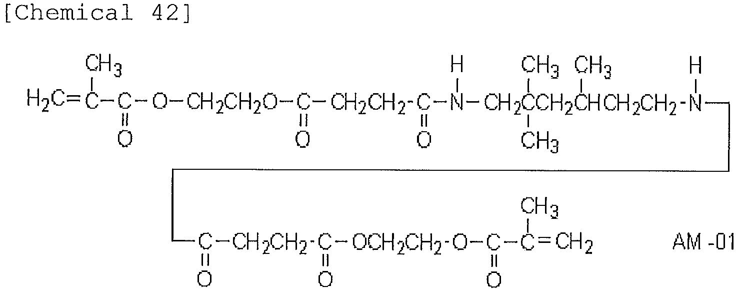 AM-02 (A1-1) : 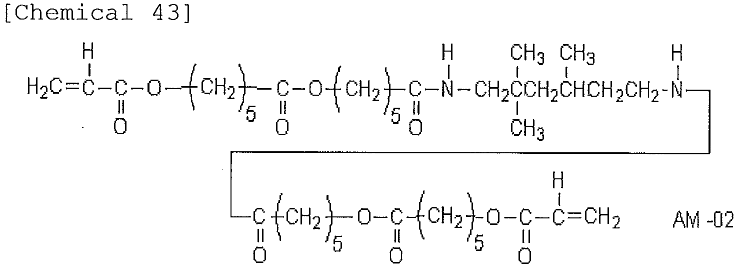 AM-03 (A1-2) : 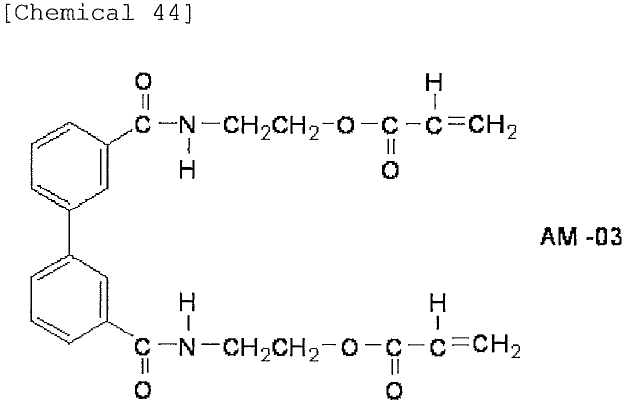 AM-04 (A1-1) : 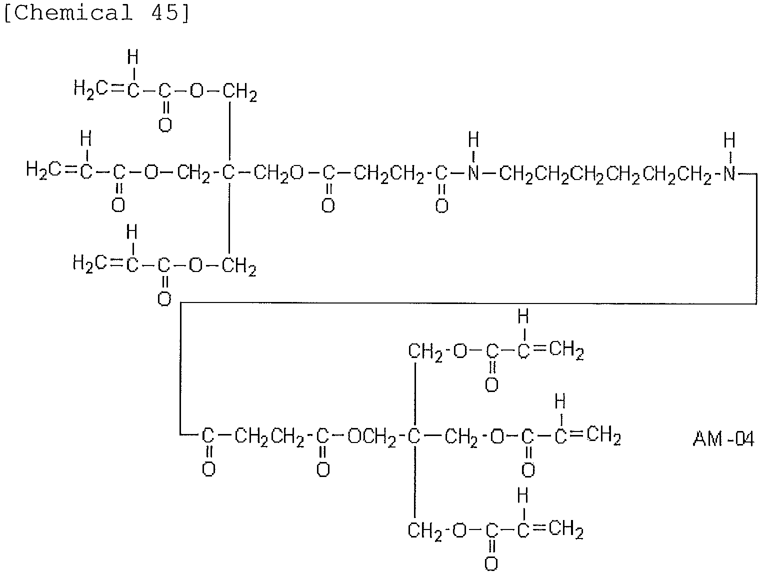 AM-05 (A1-1) : 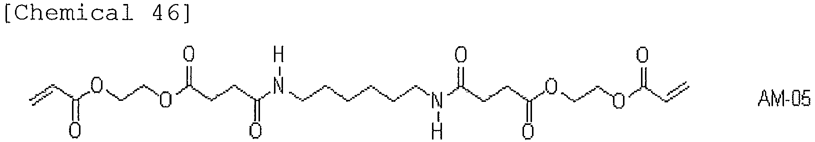 AM-06 (A1-1) : 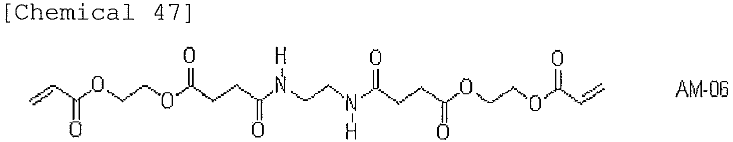 AM-07 (A1-1) : 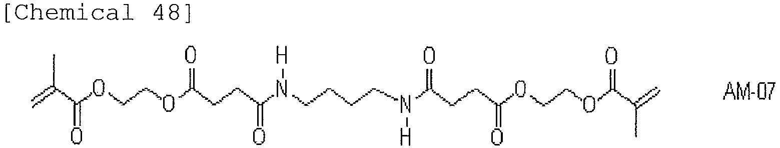 AM-08(A1-1): 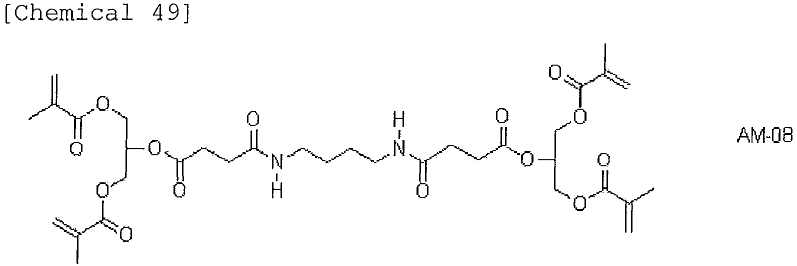 AM-09 (A1-1) : 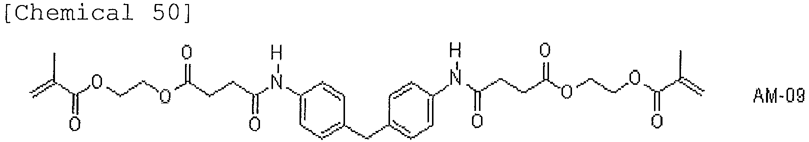 AM-10 (A1-1) : 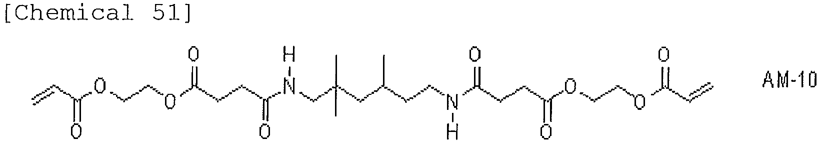 AM-11 (A1-1) :

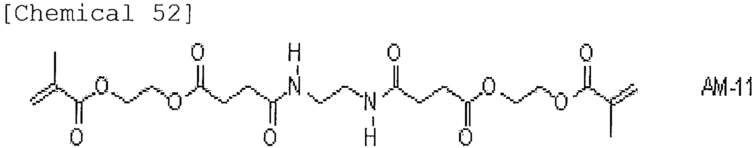

### <Production Example 1>

Production of the (meth)acrylic - amide polymerizable monomer (A1) AM-01:
β-Methacryloyloxyethylhydrogen succinate, 23.0 g (0.1 mol),
2,2,4-Trimethylhexamethylenediamine,
   7.9 g (0.05 mols), and
Dimethylaminopyridine, 12.2 g (0.1 mol),
were dissolved in 200 mL of tetrahydrofuran, and to which, 1-Ethyl-3-(3-dimethylaminopropyl) carbodiimide hydrochloride (WSC), 23.0 g (0.12 mols),
was added little by little.

After stirred overnight at room temperature, the precipitated solid material was separated by filtration. The filtrate was concentrated under reduced pressure and was refined by column chromatography using neutral alumina to obtain 7.0 g of a transparent viscous liquid. The yield was 24%.

The obtained viscous liquid was measured for its proton nuclear magnetic resonance spectra to find peaks of 40H based on the methyl and methylene proton near 0.5 to 4.9 ppm and peaks of 4H based on the vinyl proton of the unsaturated bond near δ5.0 to 6.0 ppm.

From the above results, it was confirmed that the isolated product was a compound represented by the above formula. Based on the infrared absorption analytical method, further, an absorption peak stemming from the amide bond was observed at 1650 cm⁻¹.

The product was elementally analyzed to be as follows:
C: 59.90%, H: 7.97%, N: 4.72%

The C₂₉H₄₆N₂O₁₀ was calculated to be as follows:
C: 59.78%, H: 7.96%, N: 4.81%

It was learned from the above that the analytical values were in good agreement with the calculated values of the C₂₉H₄₆N₂O₁₀.

From these results, it was confirmed that the isolated product was AM-01.

### <Production Example 2>

### Production of the (meth)acrylic - amide polymerizable monomer (A1) AM-02:

The operation was carried out in the same manner as in the Production Example 1 but using a γ-carboxydicaprolactone monoacrylate, 30.0 g (0.1 mol), instead of using the β-Methacryloyloxyethylhydrogen succinate to obtain 7.6 g of a transparent viscous liquid. The yield was 21%.

The obtained viscous liquid was measured for its proton nuclear magnetic resonance spectra to find peaks of 58H based on the methyl and methylene proton near 0.5 to 4.9 ppm and peaks of 6H based on the vinyl proton of the unsaturated bond near δ5.0 to 6.0 ppm.

Based on the infrared absorption analytical method, further, an absorption peak stemming from the amide bond was observed at 1650 cm⁻¹.

The product was elementally analyzed to be as follows:
C: 64.66%, H: 9.34%, N: 3.72%

The C₃₉H₆₆N₂O₁₀ was calculated to be as follows:
C: 64.79%, H: 9.20%, N: 3.87%

It was learned from the above that the analytical values were in good agreement with the calculated values of the C₃₉H₆₆N₂O₁₀.

From these results, it was confirmed that the isolated product was AM-02.

### <Production Example 3>

Production of the (meth)acrylic - amide polymerizable monomer (A1) AM-04:
Tetramethylolmethane triacrylate,
   29.8 g (0.1 mol), and
Succinic anhydride, 11.8 g (0.1 mol),
were dissolved in 500 mL of toluene, and to which,

p-Toluenesulfonic monohydrate, 190 mg (0.001 mol) was added, and the mixture was heated and refluxed for 3 hours.

The reaction solution was concentrated under reduced pressure and was refined by column chromatography using neutral alumina to obtain 37.40 g of a transparent viscous liquid. To the viscous liquid, there added,
Hexamethylenediamine, 5.8 g (0.05 mols), and
Dimethylaminopyridine, 12.2 g (0.1 mol), the mixture thereof was dissolved in 200 mL of the tetrahydrofuran, and to which,
1-Ethyl-3-(3-dimethylaminopropyl) carbodiimide hydrochloride (WSC), 23.0 g (0.12 mols),
was added little by little.

After stirred overnight at room temperature, the precipitated solid material was separated by filtration. The filtrate was concentrated under reduced pressure and was refined by column chromatography using neutral alumina to obtain 7.9 g of a transparent viscous liquid. The yield was 18%.

The obtained viscous liquid was measured for its proton nuclear magnetic resonance spectra to find peaks of 36H based on the methyl and methylene proton near 0.5 to 4.9 ppm and peaks of 18H based on the vinyl proton of the unsaturated bond near δ5.0 to 6.0 ppm.

Based on the infrared absorption analytical method, further, an absorption peak stemming from the amide bond was observed at 1650 cm⁻¹.

The product was elementally analyzed to be as follows:
C: 57.40%, H: 6.60%, N: 3.08%

The C₄₂H₅₆N₂O₁₈ was calculated to be as follows:
C: 57.53%, H: 6.44%, N: 3.19%

It was learned from the above that the analytical values were in good agreement with the calculated values of the C₄₂H₅₆N₂O₁₈.

From these results, it was confirmed that the isolated product was AM-04.

### <Production Example 4>

### Production of the (meth)acrylic - amide polymerizable monomer (A1) AM-05:

A carboxyl group-containing acrylate (NK ESTER A-SA, produced by Shin-Nakamura Kagaku Co.), 21.6 g (100 mmols), was dissolved in 300 ml of toluene and to which a thionyl chloride, 13.1 g (110 mmols) was added to carry out the reaction at 70°C for 2 hours.

After the reaction, excess of the thionyl chloride and toluene were distilled off to obtain an acid chloride (carboxylic acid derivative) solution.

Into a separate reaction vessel,
1,6-Hexamethylenediamine (AC-1), 5.8 g (50 mmols), Tetrahydrofurane (THF), 160 ml,
Sodium hydrogencarbonate, 8.4 g (100 mols), and
Water, 160 ml,
were added and cooled with ice. To the mixed solution, an acid chloride solution was added dropwise over 30 minutes. Thereafter, the reaction was carried out for one hour at 0 to 5°C and for another 3 hours at 20 to 25°C. The reaction solution was washed with an aqueous solution containing 10% of sodium chloride to remove the organic solvent, and was refined by the column chromatography using silica gel to obtain 17.4 g of a viscous liquid. The yield was 68%.

The obtained viscous liquid was measured for its proton nuclear magnetic resonance spectra to find peaks of 28H based on the methyl and methylene proton near 0.5 to 4.9 ppm and peaks of 6H based on the vinyl proton of the unsaturated bond near δ5.0 to 6.0 ppm.

Based on the infrared absorption analytical method, further, an absorption peak stemming from the amide bond was observed at 1650 cm⁻¹.

The product was elementally analyzed to be as follows:
C: 56.09%, H: 7.15%, N: 5.56%

On the other hand, the C₂₄H₃₆N₂ was calculated to be as follows:
C: 56.24%, H: 7.08%, N: 5.45%

It was learned from the above that the analytical values were in good agreement with the calculated values of the C₂₄H₃₆N₂.

From these results, it was confirmed that the isolated product was AM-05.

### <Production Examples 5 to 11>

(Meth)acrylic - amide polymerizable monomers (A1) shown in Table 2 were synthesized by using the starting materials shown in Table 1.

The obtained products were analyzed for their structures by using the same means as the one for confirming the structure of Production Example 1, and were confirmed to be the compounds represented by the structural formulas shown in Table 2.

Table 3 shows elementally analyzed values, values calculated from the structural formulas, and characteristic ¹H-NMR spectra.

**Table 1**

| Production Example No. | Carboxyl group-containing monomer | Carboxylic acid derivative (-X) | Amine compound | Basic substance | Reaction solvent |
|---|---|---|---|---|---|
| 5 | CM-3 | -Cl | AC-1 | sodium hydrogencarbonate | toluene/water |
| 6 | CM-1 | -Cl | AC-3 | sodium hydrogencarbonate | diethyl ether/water |
| 7 | CM-2 | -Cl | AC-4 | sodium hydrogencarbonate | dimethoxyethane/water |
| 8 | CM-4 | -Cl | AC-4 | sodium hydrogencarbonate | THF/water |
| 9 | CM-2 | -Cl | AC-5 | potassium hydrogencarbonate | toluene/water |
| 10 | CM-1 | -Cl | AC-2 | sodium hydroxide | toluene/water |
| 11 | CM-1 | -Cl | AC-3 | sodium hydrogencarbonate | THF/water |

The carboxyl acid derivative used in Production Examples 5 to 11 can be synthesized from the carboxyl group-containing monomer as described above. The carboxyl group-containing monomer may be the one placed in the market or can be synthesized from the hydroxyl group-containing monomer. Described below are formulas of the carboxyl group-containing monomers used in the present invention.

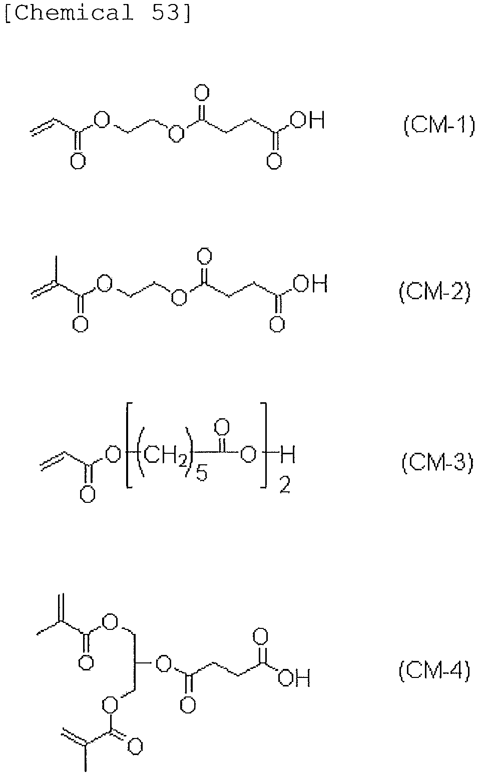

The monomers of the formulas (CM-1) and (CM-2) were purchased from Shin-Nakamura Kagaku Co. (Production names: NK ESTER A-SA and NK ESTER SA).

The monomer of the formula (CM-3) was purchased from Toa Gosei Co. (Production name: ARONIX M 5300).

The monomer of the formula (CM-4) was obtained by reacting a glycerin dimethacrylate (production name: NK ESTER 701, produced by Shin-Nakamura Kagaku Co.) with a succinic anhydride in the presence of a 4-(N,N-dimethylamino) pyridine.

The amine compounds used in Production Examples 5 to 11 can be those placed in the market. Described below are the amine compounds used in the Production Examples.
(AC-1): 1,6-hexamethylenediamine,
(AC-2): 2,2,4-trimethylhexamethylenediamine,
(AC-3): ethylenediamine,
(AC-4): 1,4-butanediamine,
(AC-5): 4,4'-diaminodiphenylmethane.

**Table 2**

| Production Example No. | | Formed product | Yield (%) |
|---|---|---|---|
| 5 | AM-02 | 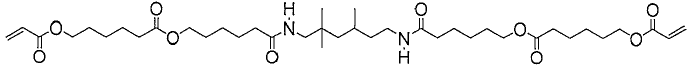 | 72 |
| 6 | AM-06 | 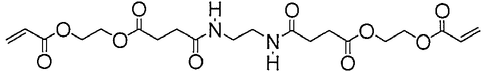 | 66 |
| 7 | AM-07 | 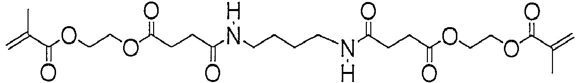 | 71 |
| 8 | AM-08 | 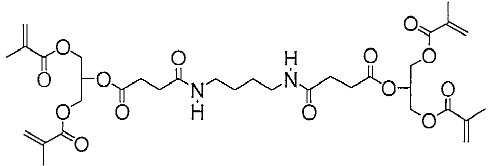 | 59 |
| 9 | AM-09 | 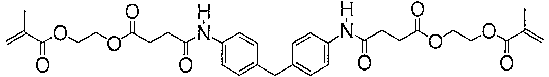 | 63 |
| 10 | AM-10 | 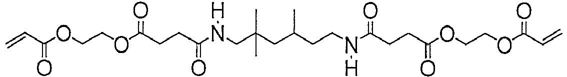 | 54 |
| 11 | AM-11 | 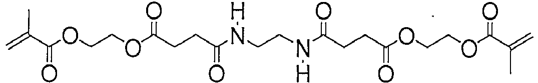 | 73 |

**Table 3**

| Production Example No. | | Elementary analyzed values | | | | | | ¹H-NMR(NMR) |
|---|---|---|---|---|---|---|---|---|
| | | Found | | | Calculated | | | |
| | | C | H | N | C | H | N | |
| 5 | AM-02 | 64.71 | 9.30 | 3.94 | 64.79 | 9.20 | 3.87 | δ5.0-6.0 6H |
| | | | | | | | | δ0.5-4.9 58H |
| 6 | AM-06 | 52.70 | 6.13 | 5.99 | 52.63 | 6.18 | 6.14 | δ5.0-6.0 6H |
| | | | | | | | | δ0.5-4.9 20H |
| 7 | AM-07 | 56.44 | 7.01 | 5.31 | 56.24 | 7.08 | 5.47 | δ5.0-6.0 4H |
| | | | | | | | | δ0.5-4.9 30H |
| 8 | AM-08 | 57.56 | 6.93 | 3.84 | 57.62 | 6.83 | 3.95 | δ5.0-6.0 8H |
| | | | | | | | | δ0.5-4.9 38H |
| 9 | AM-09 | 63.67 | 6.18 | 4.75 | 63.65 | 6.15 | 4.50 | δ8.0-6.1 4H |
| | | | | | | | | δ5.0-6.0 4H |
| | | | | | | | | δ0.5-4.9 28H |
| 10 | AM-10 | 58.43 | 7.76 | 4.96 | 58.47 | 7. 63 | 5.05 | δ5.0-6.0 6H |
| | | | | | | | | δ0.5-4.9 34H |
| 11 | AM-11 | 54.78 | 6.88 | 5.95 | 54.54 | 6.66 | 5.78 | δ5.0-6.0 4H |
| | | | | | | | | δ0.5-4.9 26H |

### <Non-amide polymerizable monomers (A2)>

A radically polymerizable monomer having at least three radically polymerizable groups but without having amide bond;
TMPT: trimethylolpropane trimethacrylate
MA0735: a mixture containing three kinds of the compounds having structures represented by the following formulas (MA0735(1)), (MA0735(2)) and (MA0735(3)) at a ratio of about 1:1:1.

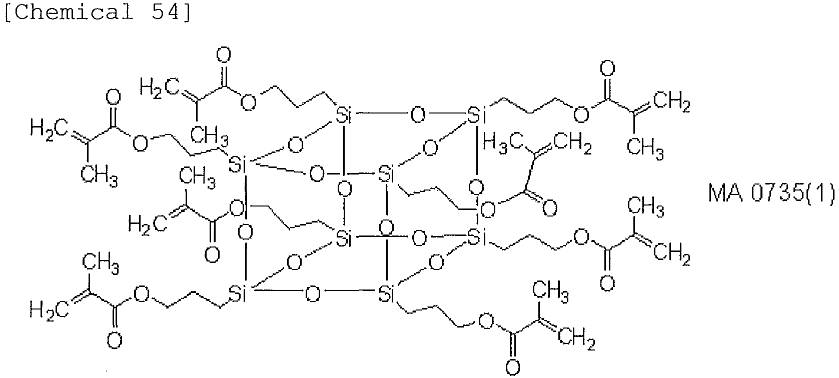

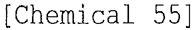

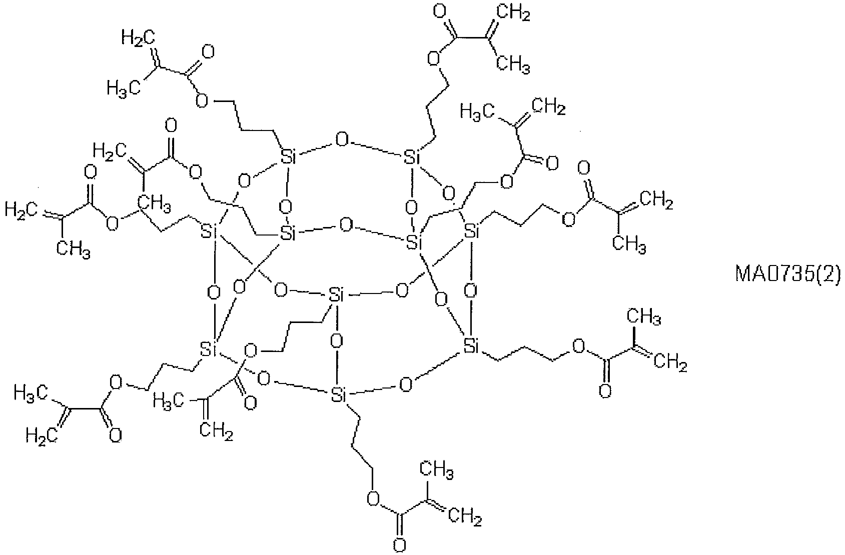

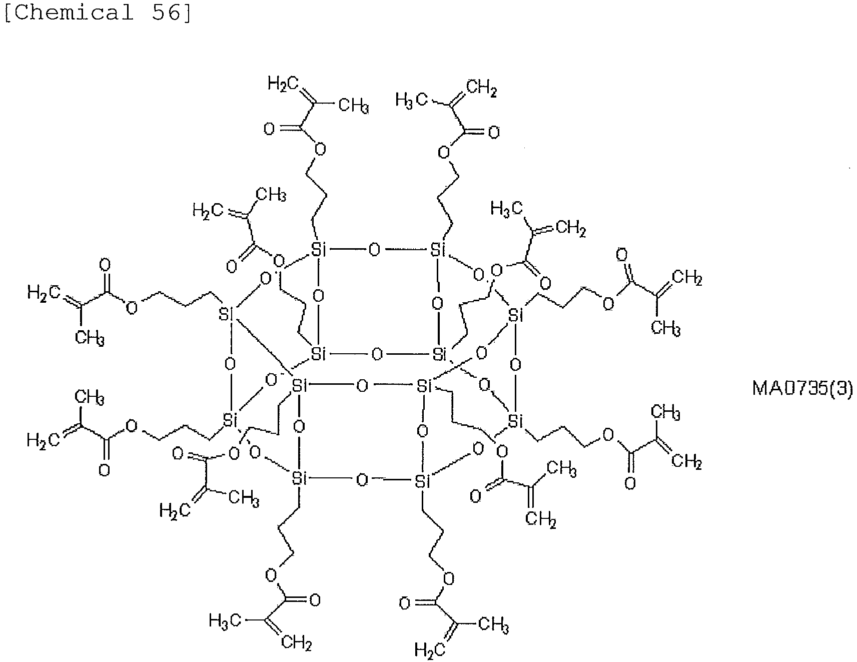

### OL1160:

A compound represented by the following formula (OL1160)

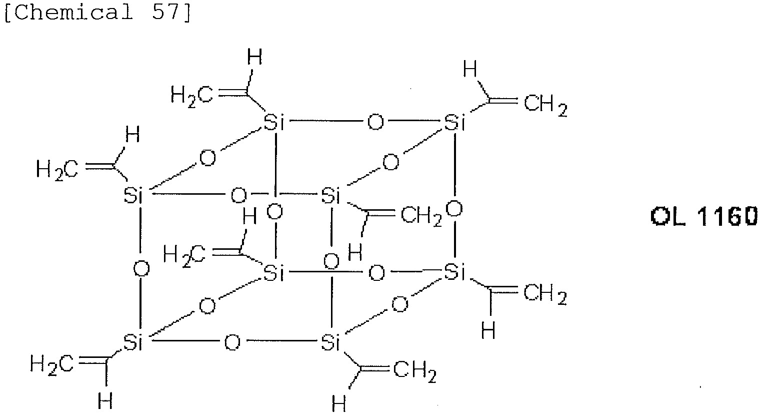

### <Photochromic compounds (B)>

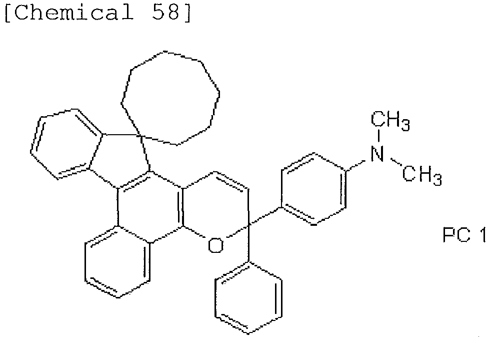

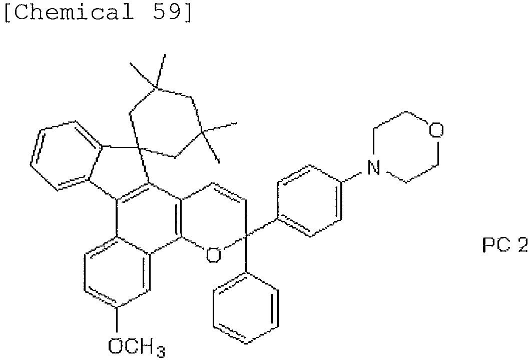

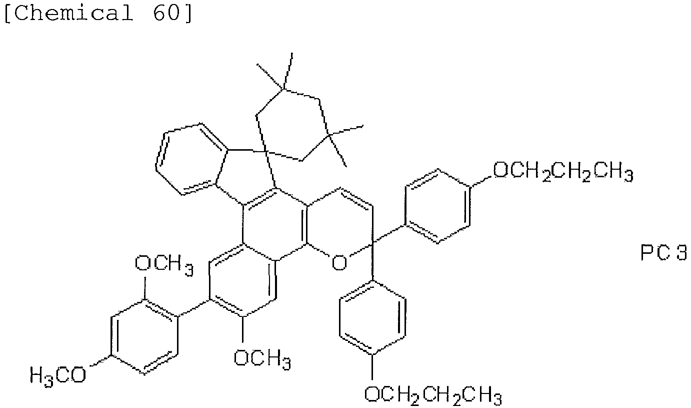

### <Other non-amide polymerizable monomers (A3)>

BPE100: 2,2-bis(4-methacryloxypolyethoxyphenyl) propane (average length of ethylene glycol chain, 2.6; average molecular weight, 478)
BPE500: 2,2-bis(4-methacryloyloxypolyethoxyphenyl) propane (average length of ethylene glycol chain, 10; average molecular weight, 804)
3PG: tripropylene glycol dimethacrylate
4G: Tetraethylene glycol dimethacrylate
9G: Polyethylene glycol dimethacrylate (average length of ethylene glycol chain, 9; average molecular weight, 536)
A-BPE: 2,2-bis(4-acryloyloxypolyethoxyphenyl) propane (average length of ethylene glycol chain, 10; average molecular weight, 776)
9GA: polyethylene glycol diacrylate (average length of ethylene glycol chain, 9; average molecular weight, 508)
MePEGMA: methyl ether polyethylene glycol methacrylate (average length of ethylene glycol chain, 23; average molecular weight, 1068) (homo HL < 20)
GMA: glycidyl methacrylate (molecular weight, 142) (homo HL = 80)
αMS: α-methylstyrene
MSD: α-methylstyrene dimer
MMS: 3-methacryloxypropyltrimethoxysilane

### <Other blending agents>

### (Polymerization initiators)

Perbutyl ND: t-butylperoxyneodecanoate
Perocta O: 1,1,3,3-tetramethylbutylperoxy-2-ethyl hexanoate
CGI1800: a mixture of 1-hydroxycyclohexylphenylketone and bis(2,6-dimethoxybenzoyl-2,4,4-trimethyl-pentyl phosphinoxide (weight ratio: 3 to 1)

### (Hindered amine photo stabilizer)

Tinuvin 765: bis(1,2,2,6,6-pentamethyl-4-piperidyl) sebacate

### (Hindered phenol antioxidant)

IRGANOX 245: ethylene bis(oxyethylene)bis[3,5-tert-butyl-4-hydroxy-m-toluyl] propionate

### <Example 1>

AM-01 (polymerizable monomer A1), 50 parts by mass,
TMPT (polymerizable monomer A2), 20 parts by mass,
9GA (polymerizable monomer A3), 25 parts by mass,
GMA (polymerizable monomer A3), 1 part by mass,
αMS (polymerizable monomer A3), 3 parts by mass, and
MSD (polymerizable monomer A3), 1 part by mass, were mixed well, and in which,
PC1 (photochromic compound B), 0.04 parts by mass, was dissolved, and to which were, further, added as polymerization initiators,
perbutyl ND, 1 part by mass, and
perocta O, 0.1 part by mass,
followed by mixing to a sufficient degree.

The mixed liquid (photochromic curable composition) was poured into a mold constituted by glass plates and gaskets of an ethylene-vinyl acetate copolymer, and was mold-polymerized. The polymerization was carried out in an air furnace by gradually elevating the temperature to 30°C through up to 90°C over 18 hours and holding the temperature at 90°C for 2 hours. After the polymerization, the polymer was taken out from the glass mold.

A sample of the obtained photochromic cured body (2 mm thick) was irradiated with light from a xenon lamp, L-2480 (300W) SHL-100, manufactured by Hamamatsu Photonics Co. through an aeromass filter (manufactured by Corning Co.) under the following conditions for 120 seconds to develop color and to measure the photochromic properties of the cured body.
Temperature: 20°C ± 1°C
Beam intensities (on the surface of the polymer):
   365 nm = 2.4 mW/cm²,
   245 nm = 24 µW/cm²

The photochromic properties were evaluated based on maximum absorption wavelengths, color densities and fading rates measured by the methods described below.

### Maximum absorption wavelengths (λmax):

A maximum absorption wavelength after color has been developed was found by using a spectrophotometer {instantaneous multi-channel photo detector MCPD1000} manufactured by Otsuka Denshi Kogyo Co.

The maximum absorption wavelength is dependent on a color tone of when the color is being developed. Color density {ε(120) - ε(0)}:

The color density was found as a difference between the absorbency ε(0) and the absorbency {ε(120)} at the maximum absorption wavelength after irradiated with light for 120 seconds.

The higher the value the more excellent the photochromic properties are. Further, the color tone of color developed outdoors was evaluated with the eyes.

### Fading rate [t1/2 (sec)]:

After the photochromic cured body was irradiated with light for 120 seconds, irradiation of light was discontinued, and the time was measured until the absorbency of the cured body at the maximum wavelength decreased down to one-half the value of {ε(120) - ε(0)}.

The shorter the time the more excellent the photochromic properties are.

The photochromic cured body was, further, measured for its tensile strength and refractive index by the methods described below.

Table 4 shows the photochromic cured composition and Table 5 shows the evaluated results thereof.

### Tensile strength (kgf):

A disk-like test piece 2 mm in thickness and 5 cm φ in diameter was prepared from the cured body obtained above.

Two holes 2 mm φ in diameter were perforated by drilling in the disk-like test piece on a line of the diameter of the disk-like test piece at points 4 mm away from the circumference thereof.

Stainless steel rods 1.6 mm φ in diameter were passed through the two holes, respectively. The two rods passing through the test piece were fixed to the upper and lower chucks of a tension tester, and the tension test was conducted at a rate of 5 mm/min.

The mechanical strength was evaluated based on the tensile strength at this moment.

The CR-39 which is a typical material used as the spectacle lenses can be measured for its tensile strength to be 18 kgf. If the value is less than 12 kgf, the material is not strong enough when it is used as spectacle lenses.

### Refractive index:

A refractive index was measured at 20°C by using a refractometer manufactured by Atago Co. By using a bromonaphthaline or a methylene iodide as the contact solution, a refractive index was measured on the D-line.

### <Examples 2 to 10>

By preparing photochromic curable compositions shown in Table 4 in the same manner as in Example 1, lenses were prepared and evaluated in the same manner as in Example 1. Table 4 shows the curable compositions and Table 5 shows the evaluated results.

**Table 4**

| *1 | *2 | *3 | *4 | *5 | *6 | *7 | *8 |
|---|---|---|---|---|---|---|---|
| 1 | AM-01 (50) | TMPT (20) | 40 | 9GA (25)/GMA (1)/ αMS (3) /MSD (1) | PC1(0.05) | - | ND (1) O(0.1) |
| 2 | AM-01 (30) | TMPT (10) | 33 | BPE100(40)/9GA (10)/GMA (1)/ αMS (8) /MSD (1) | PC1(0.05) | - | ND (1) O(0.1) |
| 3 | AM-01 (40) | TMPT (10) | 25 | 3PG(35)/9GA(10)/GMA(3)/ MSD(2) | PC1(0.05) | - | ND (1) O(0.1) |
| 4 | AM-01 (35) | MA0735 (15) | 43 | 3PG(25)/4G(22)/GMA(1)/ MSD (2) | PC1(0.05) | - | ND (1) O(0.1) |
| 5 | AM-02 (15) | TMPT (7) | 47 | BPE100(20)/4G(35)/9GA(10)/GMA(3)/ αMS(8)/MSD(2) | PC1(0.05) | - | ND (1) O(0.1) |
| 6 | AM-02 (20) | OL1660 (20) | 100 | BPE100(45)/9GA(10)/GMA(1)/ αMS(2)/MSD(2) | PC2(0.05) | - | ND (1) O(0.1) |
| 7 | AM-03 (25) | TMPT (10) | 40 | BPE100(45)/9GA(10)/GMA(1)/ αMS(8)/MSD(1) | PC2(0.05) | - | ND (1) O(0.1) |
| 8 | AM-03 (50) | MA0735 (15) | 30 | 4G(25)/MePEGMA(8)/GMA(1)/ MSD(1) | PC3(0.1) | - | ND (1) O(0.1) |
| 9 | AM-04 (40) | TMPT (5) MA0735 (15) | 50 | 3PG (20) /A-BPE(15)/GMA(1)/ αMS(3)/MSD(1) | PC2(0.05) | - | ND (1) O(0.1) |
| 10 | AM-04 (20) | MA0735 (25) | 120 | BPE100(40)/9GA(10)/GMA(1)/ αMS (2)/MSD (2) | PC3(0.1) | - | ND (1) O(0.1) |

| | | | | | | | |
|---|---|---|---|---|---|---|---|
| *1: Example No., *2: Component (A1) (mass parts), *3: Component (A2) (mass parts), *4: Mass parts of A2 per 100 mass parts of A1, *5: Component (A3) (mass parts), *6: Component (B) (mass parts), *7: Additive (mass parts), *8: Initiator (mass parts) | | | | | | | |

**Table 5**

| *1 | *2 | *3 | *4 | *5 | *6 |
|---|---|---|---|---|---|
| 1 | 588nm | 1.3 | 40 | 30 | 1.50 |
| 2 | 588nm | 1.2 | 46 | 32 | 1.54 |
| 3 | 588nm | 1.2 | 51 | 30 | 1.50 |
| 4 | 588nm | 1.3 | 40 | 37 | 1.50 |
| 5 | 588nm | 1.1 | 46 | 30 | 1.53 |
| 6 | 578nm | 1.2 | 36 | 34 | 1.55 |
| 7 | 578nm | 1.1 | 42 | 32 | 1.56 |
| 8 | 450nm | 0.9 | 38 | 35 | 1.50 |
| | 572nm | 0.9 | | | |
| 9 | 578nm | 1.1 | 38 | 35 | 1.50 |
| 10 | 450nm | 0.9 | 40 | 28 | 1.54 |
| | 572nm | 0.9 | | | |

| | | | | | |
|---|---|---|---|---|---|
| *1: Example No. *2: Maximum absorption wavelength (λmax) *3: Color density(Abs.) *4: Fading rate (seconds) *5: Tensile strength(kgf) *6: Refractive index | | | | | |

### <Comparative Examples 1 and 2>

By preparing photochromic curable compositions shown in Table 6, lenses were prepared and evaluated in the same manner as in Example 1. Table 6 shows the curable compositions and Table 7 shows the evaluated results.

**Table 6**

| *1 | *2 | *3 | *4 | *5 | *6 | *7 |
|---|---|---|---|---|---|---|
| 1 | - | TMPT (20) | 3PGX(50)/9GA(25)/GMA(1)/ αMS(3)/MSD(1) | PC1 (0.05) | - | ND (1) O(0.1) |
| 2 | AM-01 (50) | - | 3PGX(20)/9GA(25)/GMA(1)/ αMS(3)/MSD(1) | (0.05) | - | ND(1) O(0.1) |

| | | | | | | |
|---|---|---|---|---|---|---|
| *1: Comparative Example No. *2: Component (A1) (mass parts) *3: Component (A2) (mass parts) *4: Component (A3) (mass parts) *5: Component (B) (mass parts) *6: Additive (mass parts) *7: Initiator (mass parts) | | | | | | |

**Table 7**

| *1 | *2 | *3 | *4 | *5 | *6 |
|---|---|---|---|---|---|
| 1 | 588nm | 1.3 | 52 | 10 | 1.50 |
| 2 | 588nm | 1.0 | 94 | 38 | 1.50 |

| | | | | | |
|---|---|---|---|---|---|
| *1: Comparative Example No. *2: Maximum absorption wavelength (λmax) *3: Color density(Abs.) *4: Fading rate (seconds) *5: Tensile strength(kgf) *6: Refractive index | | | | | |

Comparative Example 1 did not use the (meth)acrylic - amide polymerizable monomer (A1). The cured body prepared by using the photochromic curable composition of this case exhibited a low tensile strength.

Comparative Example 2 did not use the non-amide polymerizable monomer (A2). The cured body prepared by using the photochromic curable composition of this case exhibited a low color density and a slow fading rate.

### <Example 11>

AM-02 (polymerizable monomer A1), 40 parts by mass,
MA0735 (polymerizable monomer A2), 30 parts by mass,
9G (polymerizable monomer A3), 29 parts by mass, and
GMA (polymerizable monomer A3), 1 part by mass,
were mixed well, and to which PC1 (photochromic compound B) was added in an amount of 2 parts by mass, followed by mixing to a sufficient degree to obtain a photochromic curable composition.

To the composition were, further, added,
TINUVIN 765 (photo stabilizer), 5 parts by mass,
IRGANOX 245 (antioxidant), 3 parts by mass, and
CGI 1800 (polymerization initiator), 0.5 parts by mass,
followed by mixing to a sufficient degree to obtain a photochromic coating agent.

As the optical material, on the other hand, there was prepared a thiourethane plastic lens (thickness: 2 mm, refractive index = 1.60).

By using a spin coater, 1H-DX2, manufactured by MIKASA Co., a moisture-curable primer (TR-SC-P manufactured by Tokuyama Co.) was applied onto the surface of the plastic lens at 70 rpm for 15 seconds and at 1000 rpm for another 10 seconds.

Further, by using the above spin coater, about 2 g of the photochromic coating agent obtained as described earlier was applied onto the surface of the above surface-treated plastic lens at 60 rpm for 40 seconds and at 600 rpm for another 10 to 20 seconds to form a coating of a thickness of 40 µm.

The thus coated lens was irradiated with light from a metal halide lamp of an output of 200 mW/cm² for 90 seconds in a nitrogen gas atmosphere to cure the coating and was, further, heated at 110°C for one hour to obtain a photochromic lens forming a photochromic coating thereon.

A sample of the obtained photochromic coated lens was measured for its maximum absorption wavelength, color density and fading rate according to the methods described above. To evaluate the mechanical strength (film strength) of the cured body further, the sample was measured for its Vickers' hardness by the method described below.

Table 8 shows the composition of the photochromic coating agent and Table 9 shows the evaluated results.

Vickers' hardness: By using a hardness meter equipped with an automatic meassuring (reading) device, PMT-X7A, manufactured by Matsuzawa Co., the Vickers' indentater was pushed into the above lens having the photochromic coating with 10 gf for 30 seconds, and its Vickers' hardness was found from the indentation.

### <Examples 12 to 15>

By preparing photochromic coating agents of the compositions shown in Table 8 in the same manner as in Example 11 and by using these coating agents, photochromic lenses were prepared and evaluated in the same manner as in Example 11. Table 9 shows the evaluated results.

**Table 8**

| *1 | *2 | *3 | *4 | *5 | *6 | *7 | *8 |
|---|---|---|---|---|---|---|---|
| 11 | AM-02 (40) | MA0735 (30) | 75 | 9G(29)/GMA(1) | PC1 (2) | TINUVIN 765(5) | CGI1800 (0.5) |
| | | | | | | IRGANOX 245(3) | |
| 12 | AM-02 (30) | TMPT (30) | 100 | A-BPE(20)/9G(19)/GMA(1) | PC1 (2) | TINUVIN 765(5) | CGI1800 (0.5) |
| | | | | | | IRGANOX 245(3) | |
| 13 | AM-01 (35) | MA0735 (30) | 86 | 9G(34)/GMA(1) | PC2 (2.5) | TINUVIN 765(5) | CGI1800 (0.5) |
| | | | | | | IRGANOX 245(3) | |
| 14 | *AM-03 (35) | TMPT (25) | 71 | BPE500(20)/9GA(19)/GMA(1) | PC3 (3) | TINUVIN 765(5) | CGI1800 (0.5) |
| | | | | | | IRGANOX 245(3) | |
| 15 | AM-04 (20) | TMPT (20) | 100 | BPE500(20)/9GA(19)/GMA(1) | PC3 (3) | TINUVIN 765(5) | CGI1800 (0.5) |
| | | MA0735 (20) | | | | IRGANOX 245(3) | |

| | | | | | | | |
|---|---|---|---|---|---|---|---|
| Note: AM-03 corresponds to the (meth)acrylic - amide polymerizable monomer (A1-2) represented by the general formula (2). *1: Example No. *2: Component (A1) (mass parts) *3: Component (A2) (mass parts) *4: Mass parts of A2 per 100 mass parts of A1 *5: Component (A3) (mass parts) *6: Component (B) (mass parts) *7: Additive (mass parts) *8: Initiator (mass parts) | | | | | | | |

**Table 9**

| Ex. No. | Maximum absorption wavelength (λmax) | Color density (Abs.) | Fading rate (seconds) | Vickers' hardness |
|---|---|---|---|---|
| 11 | 588nm | 1.3 | 38 | 8.7 |
| 12 | 588nm | 1.3 | 40 | 8.2 |
| 13 | 578nm | 1.1 | 35 | 8.8 |
| 14 | 450nm | 0.9 | 40 | 8.5 |
| | 572nm | 0.9 | | |
| 15 | 450nm | 0.9 | 36 | 8.0 |
| | 572nm | 0.9 | | |

### <Comparative Examples 3 and 4>

By preparing photochromic coating agents of the compositions shown in Table 10 in the same manner as in Example 11 and by using these coating agents, photochromic lenses were prepared and evaluated in the same manner as in Example 11. Table 11 shows the evaluated results.

**Table 10**

| *1 | *2 | *3 | *4 | *5 | *6 | *7 |
|---|---|---|---|---|---|---|
| 3 | - | MA0735 (30) | A-BPE(40)/9G(29)/GMA(1) | PC1 (2) | TINUVIN 765(5) | CGI1800 (0.5) |
| | | | | | IRGANOX 245(3) | |
| 4 | AM-02 (40) | | A-BPE(30)/9G(29)/GMA(1) | PC1 (2) | TINUVIN 765(5) | CGI1800 (0.5) |
| | | | | | IRGANOX 245(3) | |

| | | | | | | |
|---|---|---|---|---|---|---|
| *1: Comparative Example No. *2: Component (A1) (mass parts) *3: Component (A2) (mass parts) *4: Component (A3) (mass parts) *5: Component (B) (mass parts) *6: Additive (mass parts) *7: Initiator (mass parts) | | | | | | |

**Table 11**

| Comp. Ex. No. | Maximum absorption wavelength (λmax) | Color density (Abs.) | Fading rate (seconds) | Vickers' hardness |
|---|---|---|---|---|
| 3 | 588nm | 1.1 | 55 | 5.7 |
| 4 | 588nm | 1.0 | 80 | 2.7 |

Comparative Example 3 did not use the (meth)acrylic - amide polymerizable monomer (A1). The photochromic coated lens prepared by using the coating agent of this case exhibited a slightly low color density, a slightly slow fading rate and a slightly low Vickers' hardness.

Comparative Example 4 did not use the non-amide polymerizable monomer (A2). The photochromic coated lens prepared by using the coating agent of this case exhibited a low color density, a slow fading rate and a considerably low Vickers' hardness.

### <Examples 16 to 25>

By preparing photochromic coating agents of the compositions shown in Table 12 in the same manner as in Example 11 and using these coating agents, photochromic lenses were prepared and evaluated in the same manner as in Example 11. Table 13 shows the evaluated results.

**Table 12**

| *1 | *2 | *3 | *4 | *5 | *6 | *7 | *8 |
|---|---|---|---|---|---|---|---|
| 16 | AM-05 (50) | TMPT (20) | 40 | 9GA(25) /GMA(1) / αMS(3)/MSD(1) | PC1 (0.05) | - | ND (1) O(0.1) |
| 17 | AM-05 (20) | MA0735 (20) | 100 | BPE100(40)/9GA(10)/GMA(1)/ αMS(8)/MSD(1) | PC1 (0.05) | - | ND (1) O(0.1) |
| 18 | AM-06 (50) | TMPT (20) | 40 | 9GA(25)/GMA(1)/ αMS(3)/MSD(1) | PC1 (0.05) | - | ND(1) O(0.1) |
| 19 | AM-06 (20) | MA0735 (20) | 100 | BPE100(40)/9GA(10)/GMA(1)/ αMS(8)/MSD(1) | PC1 (0.05) | - | ND(1) O(0.1) |
| 20 | AM-07 (50) | TMPT (20) | 40 | 9GA(25)/GMA(1)/ αMS(3)/MSD(1) | PC1 (0.05) | - | ND (1) O(0.1) |
| 21 | AM-07 (20) | MA0735 (20) | 100 | BPE100(40)/9GA(10)/GMA(1)/ αMS(8)/MSD(1) | PC1 (0.05) | - | ND (1) O(0.1) |
| 22 | AM-08 (30) | TMPT (15) | 50 | BPE100(35)/9GA(10)/GMA(1)/ αMS(8)/MSD(1) | PC1 (0.05) | - | ND (1) O(0.1) |
| 23 | AM-09 (50) | MA0735 (15) | 30 | 4G(25)/MePEGMA(8)/GMA(1)/ MSD(1) | PC1 (0.05) | - | ND (1) O(0.1) |
| 24 | AM-10 (30) | TMPT (15) | 50 | 4G(20)/3PG(15)/BPE500(15)/ GMA(1)/αMS(3)/MSD(1) | PC1 (0.05) | - | ND(1) O(0.1) |
| 25 | AM-11 (20) | MA0735 (20) | 100 | BPE100(40)/9GA(10)/GMA(1)/ αMS(2)/MSD(2) | PC1 (0.05) | - | ND (1) O(0.1) |

| | | | | | | | |
|---|---|---|---|---|---|---|---|
| *1: Example No., *2: Component (A1) (mass parts), *3: Component (A2) (mass parts), *4: Mass parts of A2 per 100 mass parts of A1, *5: Component (A3) (mass parts), *6: Component (B) (mass parts), *7: Additive (mass parts), *8: Initiator (mass parts) | | | | | | | |

**Table 13**

| *1 | *2 | *3 | *4 | *5 | *6 |
|---|---|---|---|---|---|
| 16 | 588nm | 1.2 | 43 | 32 | 1.50 |
| 17 | 588nm | 1.2 | 48 | 31 | 1.54 |
| 18 | 588nm | 1.1 | 46 | 34 | 1.50 |
| 19 | 588nm | 1.1 | 51 | 34 | 1.54 |
| 20 | 588nm | 1.1 | 44 | 35 | 1.50 |
| 21 | 588nm | 1.1 | 49 | 33 | 1.54 |
| 22 | 588nm | 1.3 | 39 | 30 | 1.53 |
| 23 | 588nm | 1.2 | 37 | 33 | 1.51 |
| 24 | 588nm | 1.1 | 48 | 32 | 1.50 |
| 25 | 588nm | 1.0 | 53 | 35 | 1.54 |

| | | | | | |
|---|---|---|---|---|---|
| *1: Example No. *2: Maximum absorption wavelength (λmax) *3: Color density(Abs.) *4: Fading rate (seconds) *5: Tensile strength(kgf) *6: Refractive index | | | | | |

### <Examples 26 to 35>

By preparing photochromic coating agents of the compositions shown in Table 14 in the same manner as in Example 11 and using these coating agents, photochromic lenses were prepared and evaluated in the same manner as in Example 11. Table 15 shows the evaluated results.

**Table 14**

| *1 | *2 | *3 | *4 | *5 | *6 | *7 | *8 |
|---|---|---|---|---|---|---|---|
| 26 | AM-01 (30) | MA0735 (15) | 50 | BPE500(15)/9G(34)/ MMS(5)/GMA(1) | PC1 (2) | TINUVIN 765(5) IRGANOX 245(3) | CGI1800 (0.5) |
| 27 | AM-02 (30) | MA0735 (15) | 50 | BPE500(15)/9G(34)/ MMS(5)/GMA(1) | PC1 (2) | TINUVIN 765(5) IRGANOX 245(3) | CGI1800 (0.5) |
| 28 | AM-05 (30) | MA0735 (15) | 50 | BPE500(15)/9G(34)/ MMS(5)/GMA(1) | PC1 (2) | TINUVIN 765(5) IRGANOX 245(3) | CGI1800 (0.5) |
| 29 | AM-07 (30) | MA0735 (15) | 50 | BPE500(15)/9G(34)/ MMS(5)/GMA(1) | PC1 (2) | TINUVIN 765(5) IRGANOX 245(3) | CGI1800 (0.5) |
| 30 | AM-10 (30) | MA0735 (15) | 50 | BPE500(15)/9G(34)/ MMS(5)/GMA(1) | PC1 (2) | TINUVIN 765(5) IRGANOX 245(3) | CGI1800 (0.5) |
| 31 | AM-06 (20) | MA0735 (25) | 125 | BPE500(30)/A-BPE(19) MMS(5)/GMA(1) | PC1 (2) | TINUVIN 765 (5) IRGANOX 245(3) | CGI1800 (0.5) |
| 32 | AM-07 (30) | MA0735 (15) TMPT (15) | 100 | A-BPE(20)/9G(19) MMS(5)/GMA(1) | PC1 (2) | TINUVIN 765 (5) IRGANOX 245(3) | CGI1800 (0.5) |
| 33 | AM-07 (30) | MA0735 (15) TMPT (15) | 100 | MePEGMA(10)/9G(24) MMS(5)/GMA(1) | PC1 (2) | TINUVIN 765(5) IRGANOX 245(3) | CGI1800 (0.5) |
| 34 | AM-08 (20) | MA0735 (15) TMPT (15) | 150 | BPE500(20)/9GA(24) MMS (5) /GMA (1) | PC1 (2) | TINUVIN 765(5) IRGANOX 245(3) | CGI1800 (0.5) |
| 35 | AM-11 (20) | MA0735 (15) TMPT (15) | 150 | BPE500(20)/9GA(19)/ MMS(5)/GMA(1) | PC1 (2) | TINUVIN 765(5) IRGANOX 245(3) | CGI1800 (0.5) |

| | | | | | | | |
|---|---|---|---|---|---|---|---|
| *1: Example No., *2: Component (A1) (mass parts), *3: Component (A2) (mass parts), *4: Mass parts of A2 per 100 mass parts of A1, *5: Component (A3) (mass parts), *6: Component (B) (mass parts), *7: Additive (mass parts), *8: Initiator (mass parts) | | | | | | | |

**Table 15**

| Ex. No. | Maximum absorption wavelength (λmax) | Color density (Abs.) | Fading rate (seconds) | Vickers' hardness |
|---|---|---|---|---|
| 26 | 588nm | 1.4 | 38 | 7.8 |
| 27 | 588nm | 1.4 | 36 | 7.6 |
| 28 | 588nm | 1.3 | 38 | 8.0 |
| 29 | 588nm | 1.3 | 41 | 8.3 |
| 30 | 588nm | 1.4 | 34 | 7.5 |
| 31 | 588nm | 1.2 | 40 | 8.3 |
| 32 | 588nm | 1.2 | 37 | 8.2 |
| 33 | 588nm | 1.3 | 31 | 7.2 |
| 34 | 588nm | 1.3 | 39 | 8.0 |
| 35 | 588nm | 1.2 | 44 | 7.8 |

## Claims

1. A photochromic curable composition containing a radically polymerizable monomer component (A) and a photochromic compound (B), wherein said radically polymerizable monomer component (A) contains:
(A1) a (meth)acrylic - amide polymerizable monomer having 2 to 6 (meth)acryloyloxy groups and 2 to 6 divalent amide groups (-NHCO-); and
(A2) a non-amide polymerizable monomer having at least 3 radically polymerizable groups but having no amide bond.

2. The photochromic curable composition according to claim 1, wherein said (meth)acrylic - amide polymerizable monomer (A1) is represented by the following general formula (1): 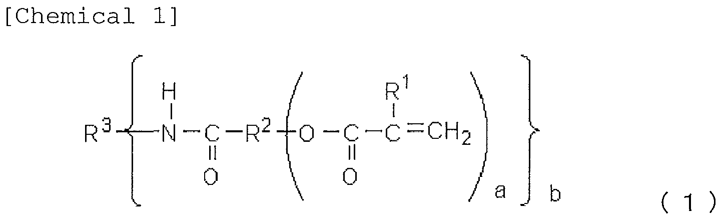 wherein,
a is an integer of 1 to 3 and b is an integer of 2 to 6 provided the product of a and b is an integer of 2 to 6,
R¹ is a hydrogen atom or a methyl group,
R² is an organic group having a valency of (a+1), and
R³ is an organic group having a valency of (b).

3. The photochromic curable composition according to claim 1, wherein said (meth)acrylic - amide polymerizable monomer (A1) is represented by the following general formula formula (2): 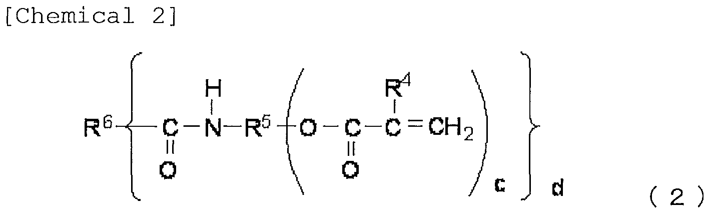 wherein,
c is an integer of 1 to 3 and d is an integer of 2 to 6 provided the product of c and d is an integer of 2 to 6,
R⁴ is a hydrogen atom or a methyl group,
R⁵ is an organic group having a valency of (c+1), and
R⁶ is an organic group having a valency of (d).

4. The photochromic curable composition according to claim 1, wherein said non-amide polymerizable monomer (A2) is a radically polymerizable monomer represented by the following general formula (3): 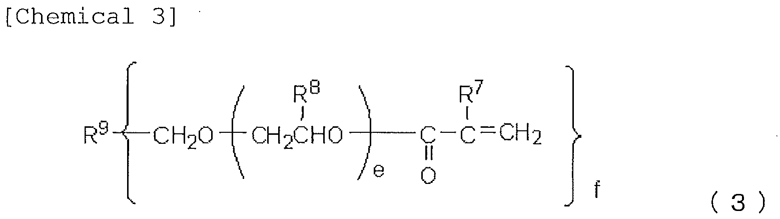 wherein,
e is an integer of 0 to 3,
f is an integer of 3 to 6,
R⁷ and R⁸ are hydrogen atoms or methyl groups, and
R⁹ is an organic group having a valency of (f).

5. The photochromic curable composition according to claim 1, wherein said non-amide polymerizable monomer (A2) has an average composition represented by the following unit formula (4):
R¹⁰SiO_{3/2} (4)
wherein,
R¹⁰ is a group bonded to a silicon atom, and is an organic group containing hydrogen atom, alkyl group, cycloalkyl group, alkoxy group, halogenated alkyl group, phenyl group, halogen-substituted phenyl group, hydroxyl group or radically polymerizable group,
and in a molecule thereof, at least 3 groups R¹⁰ are organic groups containing a radically polymerizable group, and have a polymerization degree of 6 to 100.

6. The photochromic curable composition according to claim 1, wherein said photochromic compound (B) contains a compound that has an indeno[2,1-f]naphtho [1,2-b]pyran skeleton.

7. The photochromic curable composition according to claim 1, wherein said polymerizable monomer component (A) contains the (meth)acrylic - amide polymerizable monomer (A1) in an amount of 10 to 80% by mass, the non-amide polymerizable monomer (A2) in an amount of 3 to 50% by weight and the non-amide polymerizable monomer (A3) other than the component (A2) in an amount of 0 to 87% by mass.

8. The photochromic curable composition according to claim 7, wherein said photochromic compound (B) is contained in an amount of 0.001 to 20 parts by mass per 100 parts by mass of the polymerizable monomer component (A).

9. A photochromic cured body obtained by curing the photochromic curable composition of claim 1.

10. A photochromic coating agent comprising the photochromic curable composition of claim 1.

11. A photochromic optical material obtained by forming, on the surfaces of an optical material, a photochromic coating by curing the photochromic coating agent of claim 10.

12. A (meth)acrylic - amide polymerizable monomer represented by the following general formula (1): 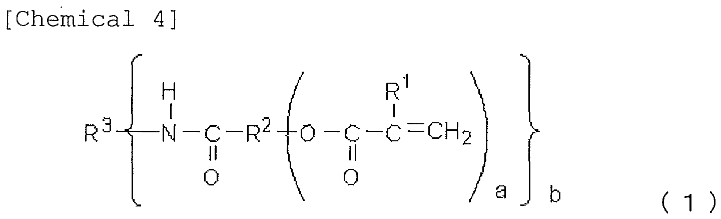 wherein
a is an integer of 1 to 3 and b is an integer of 2 to 6 provided the product of a and b is an integer of 2 to 6,
R¹ is a hydrogen atom or a methyl group,
R² is an organic group having a valency of (a+1), and
R³ is an organic group having a valency of (b).

13. The (meth)acrylic - amide polymerizable monomer according to claim 12, wherein the group R² in the general formula (1) is a group represented by the following formula (1a) or (1b), 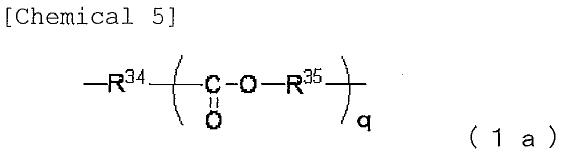 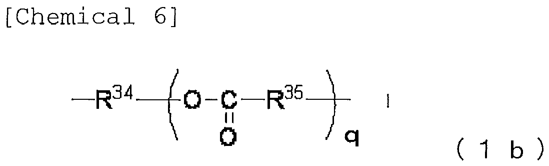 wherein in the formulas (1a) and (1b),
R³⁴ is an organic group having a valency of (a+1) that is bonded to a carboxyl oxygen atom in the (meth)acryloyloxy group, and is a group in which a saturated hydrocarbon group or a hydrogen atom in part of the saturated hydrocarbon group is substituted with an alkoxy group or a phenoxy group,
or is a group represented by the following formula (1ab-1): 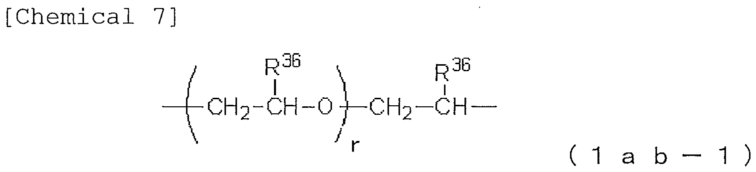
(wherein R³⁶ is a hydrogen atom or a methyl group, and r is an integer of 0 to 20),
R³⁵ is a divalent organic group, and is a saturated hydrocarbon group, an alicyclic group or an aromatic hydrocarbon group, and
q is an integer of 1 to 30.

14. The meth(acrylic - amide polymerizable monomer according to claim 12, wherein in the general formula (1), the group R³ is a group having an aromatic ring, a group having an alicyclic ring, or a saturated hydrocarbon group provided the total number of carbon atoms is 1 to 30, or these groups are oxygen-containing groups coupled to each other via
-CO-, -COO- or -O-.

15. A method of producing the (meth)acrylic - amide polymerizable monomer of claim 12 comprising reacting a carboxylic acid derivative represented by the following formula (1X): 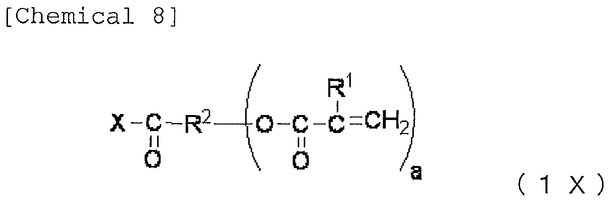 wherein,
R¹, R² and a are as defined in the above formula (1),
X is a hydroxyl group, a chlorine atom, a bromine atom, -N₃ or a group represented by the following formula:
R³⁷-COO-
(wherein R³⁷ is an alkyl group or an aryl group), with an amine compound represented by the following formula (1Y) :
R³-(NH₂)_{b} (1Y)
wherein R³ and b are as defined in the above formula (1),
in a mixed solvent of an organic solvent and water in the presence of a basic substance.
